(19) Europäisches Patentamt
European Patent Office
Office européen des brevets

(11) **EP 4 663 183 A1**

(12) **EUROPEAN PATENT APPLICATION**
published in accordance with Art. 153(4) EPC

(43) Date of publication:
**17.12.2025 Bulletin 2025/51**

(21) Application number: **24753682.4**

(22) Date of filing: **08.02.2024**

(51) International Patent Classification (IPC):
*A61K 9/08* (2006.01)     *A61K 47/22* (2006.01)
*A61K 47/26* (2006.01)     *A61K 47/18* (2017.01)
*A61K 47/12* (2006.01)     *A61K 47/02* (2006.01)
*A61K 9/19* (2006.01)     *A61K 38/17* (2006.01)
*A61P 19/08* (2006.01)     *A61P 35/00* (2006.01)

(52) Cooperative Patent Classification (CPC):
**A61K 9/08; A61K 9/19; A61K 38/17; A61K 47/02;
A61K 47/12; A61K 47/18; A61K 47/22;
A61K 47/26; A61P 19/08; A61P 35/00**

(86) International application number:
**PCT/KR2024/001962**

(87) International publication number:
**WO 2024/167361 (15.08.2024 Gazette 2024/33)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **10.02.2023 KR 20230018278**

(71) Applicant: **Gbiologics Inc.
Seongnam-si, Gyeonggi-do 13487 (KR)**

(72) Inventor: **SONG, David
Seongnam-si Gyeonggi-do 13487 (KR)**

(74) Representative: **Ström & Gulliksson AB
P.O. Box 793
220 07 Lund (SE)**

Remarks:
The complete document including Reference Table(s) and the Sequence Listing(s) can be downloaded from the EPO website

(54) **STABILIZED FORMULATION COMPRISING RECOMBINANT STABILIZED GALECTIN 9 PROTEIN**

(57) The present invention relates to a stabilized pharmaceutical formulation comprising a recombinant stabilized galectin-9 protein. More specifically, the invention provides a pharmaceutical formulation with improved stability through the combination of a buffer and a stabilizer.

Fig. 1

T0     T=45 min     T=45 min

## Description

[FIELD OF THE INVENTION]

The present invention relates to a stabilized pharmaceutical formulation comprising a recombinant stabilized galectin-9 protein. More specifically, it relates to a pharmaceutical formulation in which galectin-9 protein is stably preserved through combination with a buffer and a stabilizer.

[BACKGROUD ART]

Galectin-9 is a beta-galactoside lectin protein isolated from mouse embryonic kidney. It consists of two carbohydrate recognition domains (CRDs) and a linker peptide region connecting them, wherein the N-terminal carbohydrate recognition domain (NCRD) and the C-terminal carbohydrate recognition domain (CCRD) are linked via the linker peptide. Galectin-9 is known as one of the ligands for HAVCR2 (Tim-3), and it binds to Tim-3 to induce apoptosis of Tim-3-positive Th1 cells and suppress excessive Th1 responses, thereby alleviating autoimmune inflammation. In addition, galectin-9 has been observed in various types of cancer, including malignant melanoma, Hodgkin's lymphoma, hepatocellular carcinoma, pancreatic cancer, gastric cancer, and colorectal cancer, and efforts have been made to develop variants for therapeutic applications. In this regard, Korean Patent Publication No. 10-2022-0068158 discloses a pharmaceutical composition comprising a recombinant stabilized galectin-9 protein for the prevention or treatment of cancer. However, even as disclosed, formulation development is required to enable technical application to the human body through administration to patients.

In general, pharmaceutical formulation significantly affects the efficacy and stability of an active ingredient when applied to the human body, depending on the type and ratio of excipients used. In addition to pharmacokinetic factors such as duration of action, drug distribution, metabolism, and excretion after administration, formulation parameters must also be carefully considered in light of packaging conditions and container systems. For example, in the case of poorly soluble drugs, administration may be achieved using capsule formulations; however, depending on the manufacturing method or composition ratio, such formulations may present difficulties in administration, necessitating extensive formulation research. Furthermore, if the stability of an antibody is low, its efficacy may be compromised due to denaturation, oxidation, aggregation, or degradation, and in some cases, these phenomena may lead to adverse effects such as toxicity. Accordingly, various efforts are required to develop pharmaceutical formulations that are stable, effective, and convenient for use.

Accordingly, the present inventors have completed the present invention by conducting extensive studies on formulations for the stabilization of recombinant galectin-9 protein and identifying a formulation exhibiting enhanced stability.

[PRIOR ART DOCUMENTS]

[Patent Documents]

(Patent Document 0001) Korean Patent Publication No. 10-2022-0068158

[DETAILED DESCRIPTION OF THE INVENTION]

[PROBLEM TO BE SOLVED BY THE INVENTION]

The present invention is directed to providing a pharmaceutical formulation capable of stably preserving a recombinant stabilized galectin-9 protein.

[MEANS FOR SOLVING THE PROBLEM]

To achieve the above object, the present invention provides a pharmaceutical formulation comprising: (1) a recombinant stabilized galectin-9 protein; (2) a buffer; and (3) a stabilizer.

In one embodiment of the present invention, the recombinant stabilized galectin-9 protein may comprise an amino acid sequence represented by SEQ ID NO: 1.

In one embodiment of the present invention, the recombinant stabilized galectin-9 protein may comprise a protein having at least 90% sequence identity to the amino acid sequence represented by SEQ ID NO: 1.

In one embodiment of the present invention, the recombinant stabilized galectin-9 protein may comprise a protein in which the first amino acid from the N-terminus of the amino acid sequence of SEQ ID NO: 1 is deleted.

In one embodiment of the present invention, the recombinant stabilized galectin-9 protein may be present at a concentration of 1 to 20 mg/mL.

In one embodiment of the present invention, the buffer may be one or more selected from the group consisting of citrate, phosphate, histidine, glycine, acetate, tartrate, aspartate, lactate, gluconate, glutamate, and succinate.

In one embodiment of the present invention, the buffer may have a pH of 4.5 to 7.

In one embodiment of the present invention, the buffer may be present at a concentration of 5 to 20 mM.

In one embodiment of the present invention, the stabilizer may comprise: (i) one or more carbohydrates or sugars; and (ii) one or more amino acids or pharmaceutically acceptable salts thereof.

In one embodiment of the present invention, the carbohydrate or sugar may be selected from the group consisting of mannitol, sorbitol, xylitol, maltitol, lactitol, sucrose, trehalose, mannose, maltose, lactose, xylose, ribose, glucose, raffinose, dextran, cyclodextrin, cellobiose, isomaltose, arabinose, glucosamine, and fructose.

In one embodiment of the present invention, the carbohydrate or sugar may be selected from the group consisting of trehalose and mannitol.

In one embodiment of the present invention, the amino acid may be selected from the group consisting of glycine, alanine, serine, threonine, cysteine, valine, leucine, isoleucine, methionine, proline, phenylalanine, tyrosine, tryptophan, aspartic acid, glutamic acid, asparagine, glutamine, histidine, lysine, and arginine.

In one embodiment of the present invention, the carbohydrate or sugar comprises one or more carbohydrates or sugars, each in an amount of 1 to 5 w/v%.

In one embodiment of the present invention, the amino acid or a pharmaceutically acceptable salt thereof is present in an amount of 1 to 20 mg/mL.

In one embodiment of the present invention, the stabilizer may further comprise polysorbate.

In one embodiment of the present invention, the stabilizer further comprises polysorbate 80 in an amount of 0.001 to 0.05 w/v%.

In one embodiment of the present invention, the pharmaceutical formulation is in a liquid or lyophilized dosage form.

Furthermore, the present invention provides a pharmaceutical formulation comprising a therapeutically effective amount thereof, for use in the prevention or treatment of a bone disease.

Furthermore, the present invention provides a pharmaceutical formulation comprising a therapeutically effective amount thereof, for use in the prevention or treatment of cancer or an autoimmune disease.

Furthermore, the present invention provides a use in the manufacture of a pharmaceutical formulation for the prevention or treatment of a bone disease.

Furthermore, the present invention provides a use in the manufacture of a pharmaceutical formulation for the prevention or treatment of cancer or an autoimmune disease.

Furthermore, the present invention provides a use of a pharmaceutical formulation for the prevention or treatment of a bone disease.

Furthermore, the present invention provides a use of a pharmaceutical formulation for the prevention or treatment of cancer or an autoimmune disease.

Furthermore, the present invention provides a method for treating a bone disease, comprising administering to a subject a therapeutically effective amount of a pharmaceutical formulation.

Furthermore, the present invention provides a method for treating cancer or an autoimmune disease, comprising administering to a subject a therapeutically effective amount of a pharmaceutical formulation.

[ADVANTAGEOUS EFFECTS OF THE INVENTION]

The present invention relates to a pharmaceutical formulation comprising a recombinant galectin-9 protein, and has the advantage of providing a formulation with enhanced stability through the use of a buffer or the like.

[BRIEF DESCRIPTION OF DRAWINGS]

Fig. 1 is a diagram showing the comparative experimental results between histidine buffer and PBS buffer.

Fig. 2 is a diagram showing the comparative experimental results between acetate buffer and PBS buffer.

Fig. 3 is a diagram showing the comparative experimental results between histidine acetate buffer and sodium acetate buffer.

Figs. 4a to 4c are diagrams showing potency test results according to the pH of histidine acetate buffer.

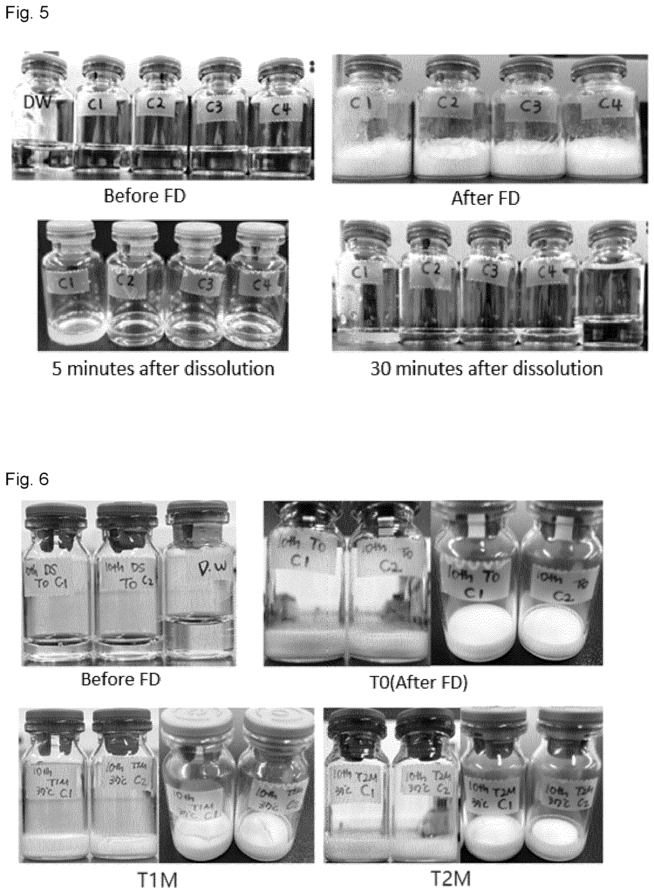

Fig. 5 is a diagram showing the appearance according to one embodiment of the present invention (Experimental Example 3-3).

Fig. 6 is a diagram showing the appearance according to one embodiment of the present invention (Experimental Example 3-6).

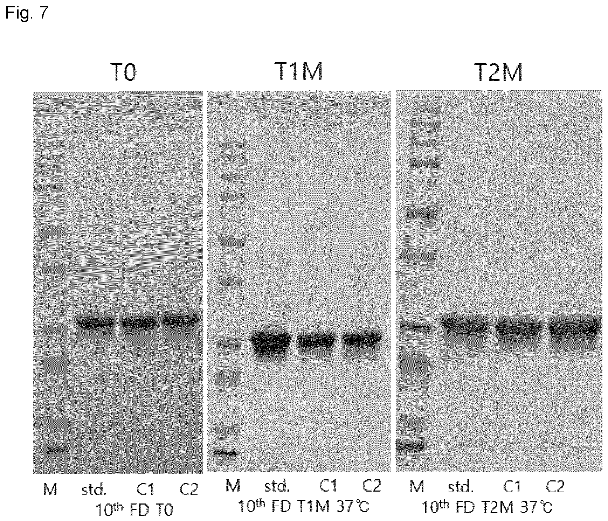

Fig. 7 is a diagram showing the SDS-PAGE analysis result according to one embodiment of the present invention (Experimental Example 3-6).

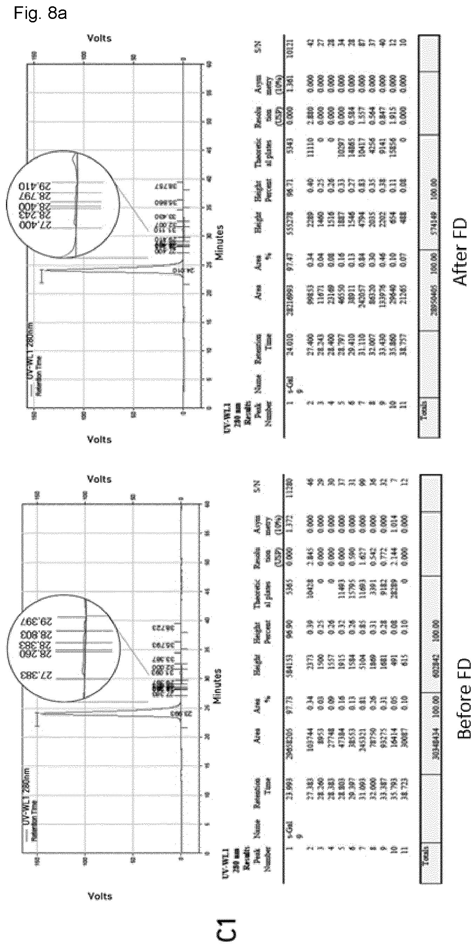

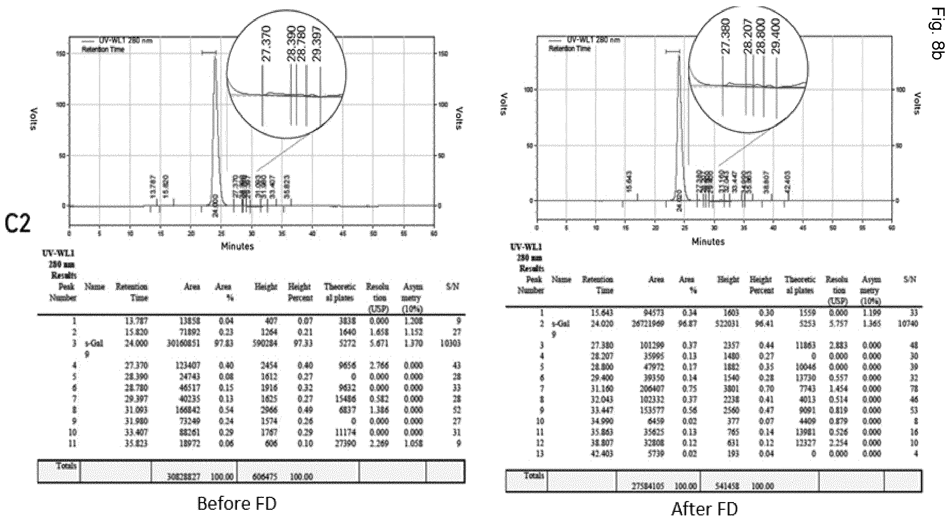

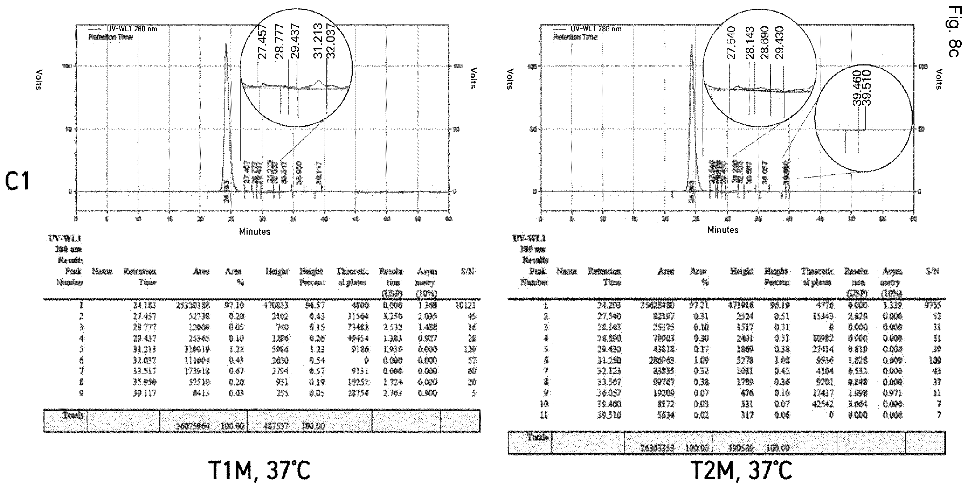

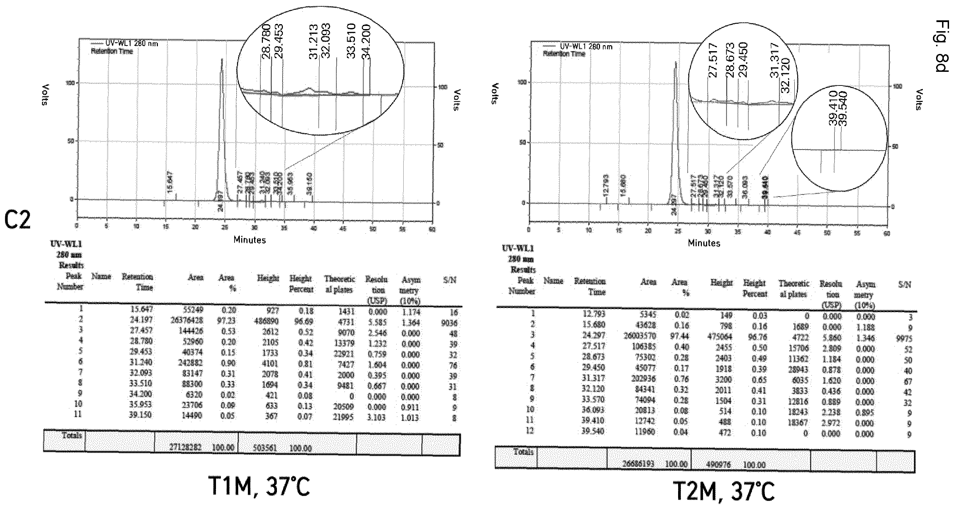

Figs. 8a to 8d are diagrams showing the SEC-HPLC analysis results according to one embodiment of the present invention (Experimental Example 3-6).

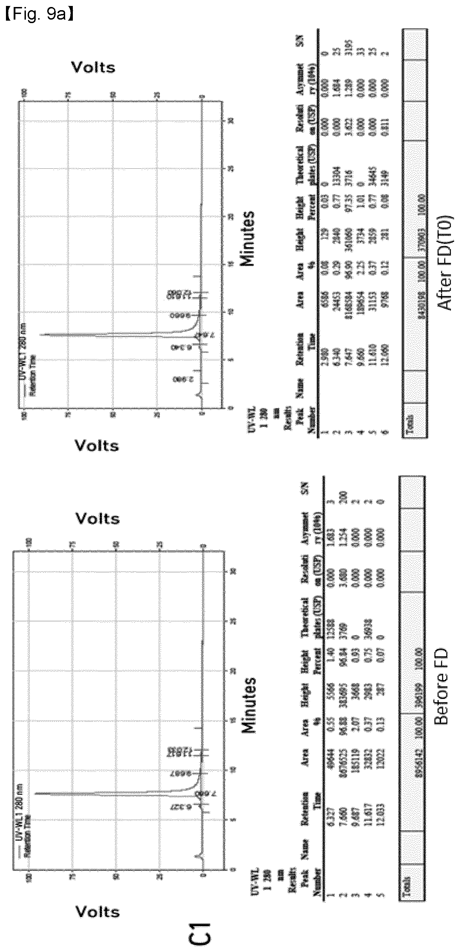

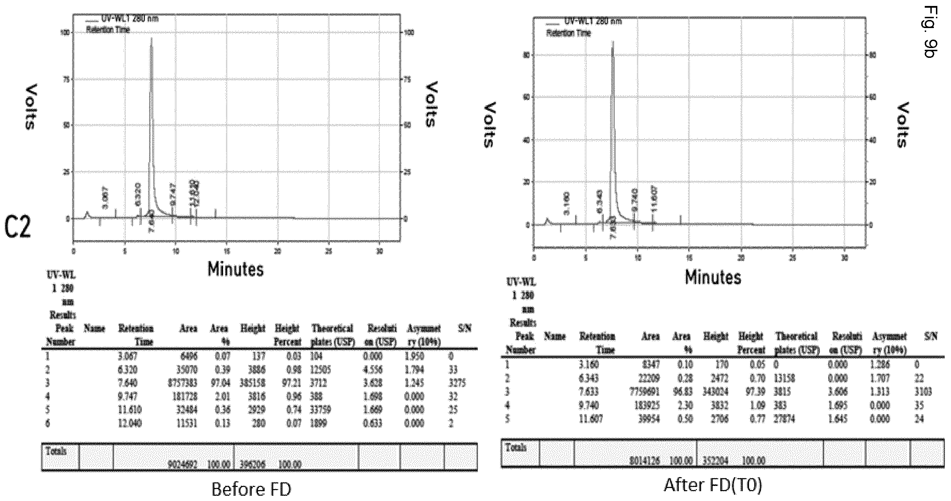

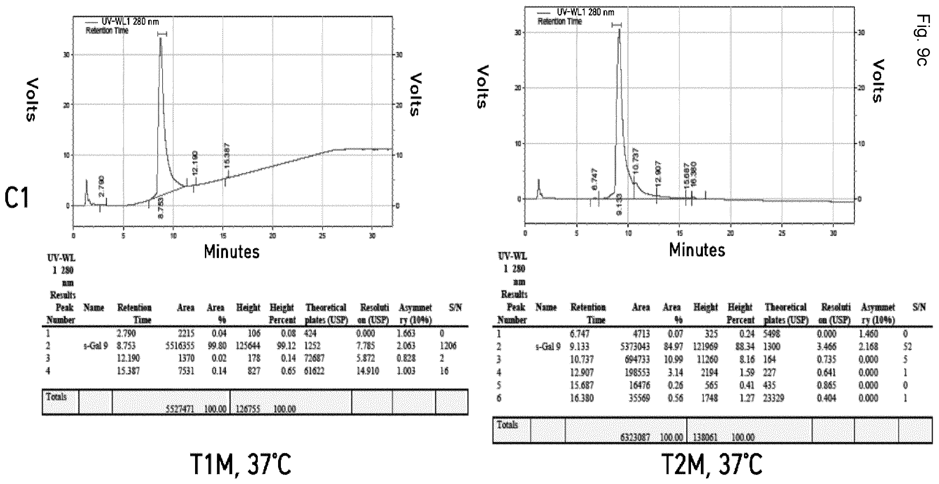

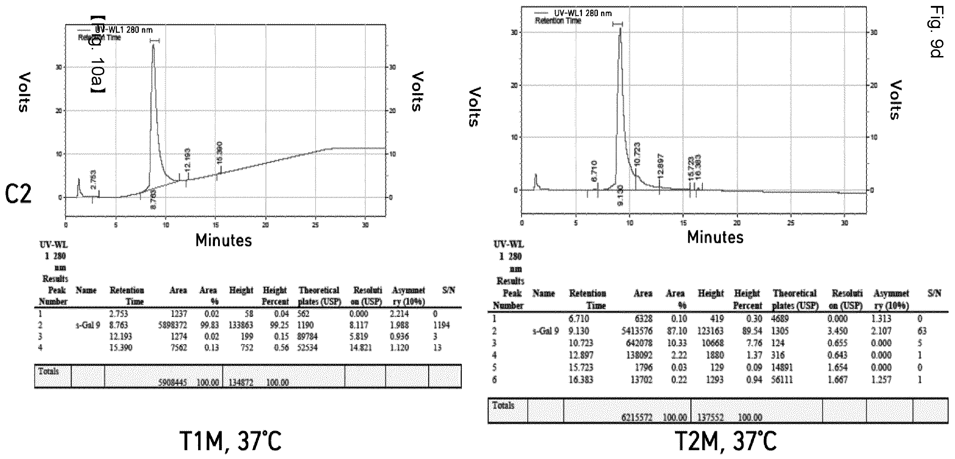

Figs. 9a to 9d are diagrams showing the IEX-HPLC analysis results according to one embodiment of the present invention (Experimental Example 3-6).

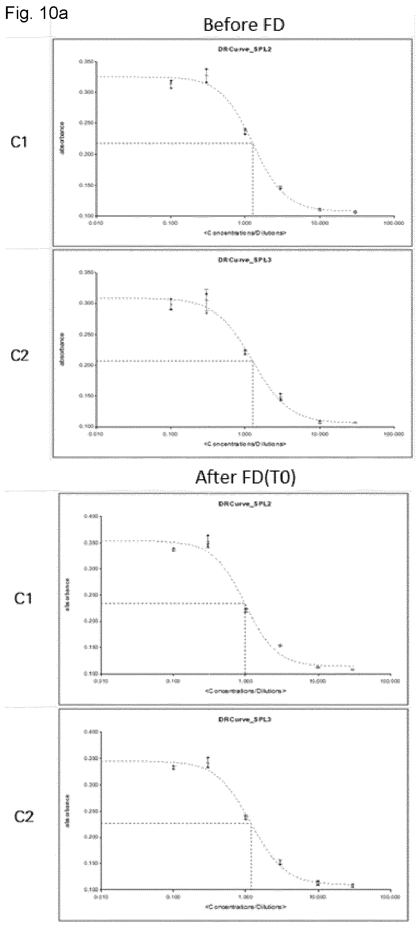

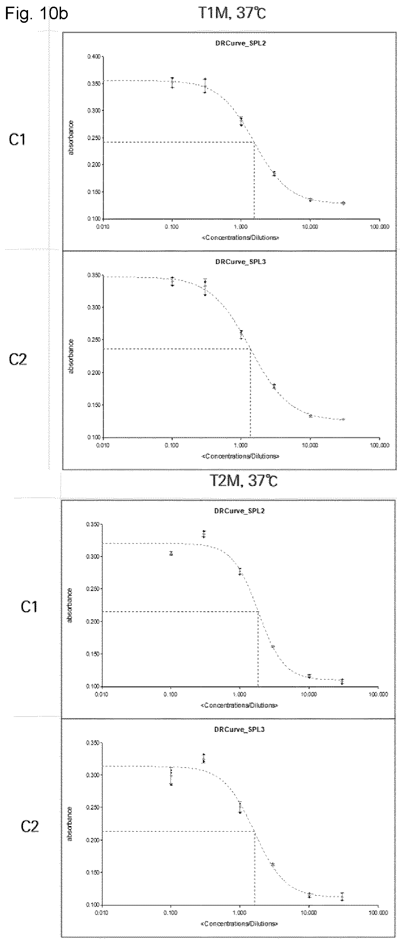

Figs. 10a to 10b are diagrams showing the potency results according to one embodiment of the present invention (Experimental Example 3-6).

[BEST MODE FOR CARRYING OUT THE INVENTION]

Hereinafter, embodiments of the present invention will be described in detail by way of example, so that those skilled in the art may implement the invention without undue difficulty. The embodiments of the present invention are provided to more completely explain the invention to those of ordinary skill in the art. Therefore, the embodiments of the present invention may be modified in various other forms, and the scope of the present invention is not intended to be limited to the embodiments described below.

It will be understood that throughout the present specification, when a component is said to "comprise" a certain element, this does not exclude the presence of other elements unless otherwise specified, but rather allows the inclusion of additional elements.

The present invention relates to a pharmaceutical formulation comprising: (1) a recombinant stabilized galectin-9 protein; (2) a buffer; and (3) a stabilizer.

In the present invention, the recombinant stabilized galectin-9 protein retains the carbohydrate recognition activity of wild-type galectin-9 while possessing a molecular structure that is more stable against proteases.

Specifically, the recombinant stabilized galectin-9 protein is a recombinant protein obtained by modifying the linker region connecting the two CRDs (Carbohydrate Recognition Domains) of wild-type galectin-9, which has a structure of NCRD-linker-CCRD, and the CCRD (C-terminal Carbohydrate Recognition Domain). More specifically, the recombinant stabilized galectin-9 protein may comprise an amino acid sequence represented by SEQ ID NO: 1, in which the peptide of the linker region is entirely deleted; an amino acid sequence (SEQ ID NO: 3) corresponding to positions 1 to 10 of the CCRD (SEQ ID NO: 2) is deleted; and the alanine (Ala; A) at position 13 is substituted with proline (Pro; P). The recombinant stabilized galectin-9 protein may comprise an amino acid sequence having at least 75%, preferably at least 80%, more preferably at least 90%, and most preferably at least 95% sequence identity to the amino acid sequence represented by SEQ ID NO: 1. In addition, the recombinant stabilized galectin-9 protein may further comprise amino acid sequences introduced for specific purposes, such as targeting sequences, tags, labeled residues, or sequences designed to increase half-life or peptide stability.

Furthermore, the recombinant protein of the present invention may be obtained by various methods well known in the art. For example, it may be produced using polynucleotide recombination and a protein expression system, synthesized in vitro by chemical synthesis such as peptide synthesis, or prepared by a cell-free protein synthesis method.

As used herein, the term "polynucleotide" refers to a polymer of nucleotides that functions to convey genetic information. For the purposes of the present invention, the polynucleotide encodes the recombinant protein of SEQ ID NO: 1, and may include a nucleotide sequence having at least 75%, preferably at least 85%, more preferably at least 90%, and most preferably at least 95% sequence identity to the polynucleotide sequence encoding said recombinant protein.

As used herein, the term "sequence identity" is intended to indicate the degree of similarity to the wild-type amino acid sequence or polynucleotide sequence. Such comparison of sequence identity may be performed using comparison programs well known in the art, and the identity between two or more sequences may be calculated as a percentage (%).

As used herein, the term "prevention" refers to any act of inhibiting or delaying the onset of a disease through the administration of the composition.

As used herein, the term "treatment" refers to any act of alleviating or favorably modifying the symptoms of a disease through the administration of the composition.

As used herein, the term "pharmaceutical formulation" refers to a final dosage form that is prepared by filling, lyophilizing, and/or reconstituting a bulk solution comprising the composition.

In one aspect of the present invention, the recombinant stabilized galectin-9 protein is a protein comprising the amino acid sequence represented by SEQ ID NO: 1.

In one aspect of the present invention, the recombinant stabilized galectin-9 protein is a protein comprising an amino acid sequence having at least 70%, at least 80%, at least 90%, at least 95%, at least 96%, at least 97%, at least 98%, or at least 99% sequence identity to the amino acid sequence represented by SEQ ID NO: 1.

In one aspect of the present invention, the recombinant stabilized galectin-9 protein comprises a deletion of the first amino acid residue from the N-terminus of the amino acid sequence represented by SEQ ID NO: 1.

In one aspect of the present invention, the recombinant stabilized galectin-9 protein may be present in the pharmaceutical composition in an amount ranging from 0.01 to 400 mg/ml. Specifically, the recombinant stabilized galectin-9 protein may be present in an amount of 0.01 to 250 mg/ml, more specifically 0.01 to 150 mg/ml, 0.01 to 120 mg/ml, 0.01 to 100 mg/ml, 0.1 to 80 mg/ml, 0.1 to 60 mg/ml, or 0.1 to 40 mg/ml. In a more specific aspect of the present invention, the recombinant stabilized galectin-9 protein may be present in an amount of 0.1 to 20 mg/ml, 0.1 to 18 mg/ml, 0.1 to 16 mg/ml, 0.1 to 15 mg/ml, 1 to 15 mg/ml, 2 to 15 mg/ml, 3 to 15 mg/ml, 4 to 15 mg/ml, or 5 to 15 mg/ml. In an even more specific aspect of the present invention, the recombinant stabilized galectin-9 protein is present in an amount of 5.5 to 15 mg/ml, 6 to 15 mg/ml, 6.5 to 15 mg/ml, 7 to 15 mg/ml, 7.5 to 15 mg/ml, 7.5 to 14.5 mg/ml, 7.5 to 14 mg/ml, 7.5 to 13.5 mg/ml, 7.5 to 13 mg/ml, or 7.5 to 12.5 mg/ml.

In one aspect of the present invention, the buffer is one or more selected from the group consisting of citrate, phosphate, histidine, glycine, acetate, tartrate, aspartate, lactate, gluconate, glutamate, succinate, and combinations thereof. In a specific aspect of the present invention, the buffer is one or more selected from the group consisting of phosphate, histidine, acetate, and combinations thereof. In a more specific aspect of the present invention, the buffer is histidine acetate. The inclusion of histidine in the present invention may provide beneficial effects in terms of stability and pH control.

In one aspect of the present invention, the buffer does not include a sodium salt.

In one aspect of the present invention, the buffer has a pH in the range of 4 to 7. Specifically, the buffer may have a pH of 4.5 to 7, more specifically 4.6 to 7, 4.7 to 7, 4.8 to 7, 4.9 to 7, or 5 to 7. In a specific aspect of the present invention, the buffer may have a pH of 5.1 to 7, 5.2 to 7, 5.3 to 7, 5.4 to 7, 5.5 to 7, 5.5 to 6.9, 5.5 to 6.8, 5.5 to 6.7, 5.5 to 6.6, or 5.5 to 6.5. In a more specific aspect of the present invention, the buffer has a pH of 5.6 to 6.5, 5.65 to 6.5, 5.6 to 6.5, 5.65 to 6.5, 5.7 to 6.5, 5.75 to 6.5, 5.75 to 6.4, or 5.75 to 6.35.

In one aspect of the present invention, the buffer in the pharmaceutical formulation may be present at a concentration of 0.01 to 50 mM. Specifically, the buffer may be present at a concentration of 0.01 to 45 mM, more specifically 0.01 to 40 mM, 0.01 to 35 mM, 0.01 to 30 mM, or 0.01 to 25 mM. In a specific aspect of the present invention, the buffer may be present at a concentration of 0.1 to 25 mM, 0.1 to 24 mM, 0.1 to 23 mM, 0.1 to 22 mM, 0.1 to 21 mM, 1 to 21 mM, 2 to 21 mM, 3 to 21 mM, or 4 to 21 mM. In a more specific aspect of the present invention, the buffer is present at a concentration of 4 to 20.5 mM, 4 to 20 mM, 4.5 to 20 mM, or 5 to 20 mM.

In one aspect of the present invention, the stabilizer comprises (i) at least one carbohydrate or sugar; and (ii) at least one amino acid or a pharmaceutically acceptable salt thereof. As used in the present invention, the term "carbohydrate or sugar" is intended to encompass monosaccharides, sugar alcohols, sugar acids, and sugar derivatives. Examples include, but are not limited to, glucose, fructose, sucrose, lactose, maltose, trehalose, glycerol, erythritol, arabitol, xylitol, mannitol, sorbitol, galactitol, fucitol, iditol, maltitol, lactitol, maltotriitol, and maltotetraitol.

As used in the present invention, the amino acid may include natural amino acids or non-natural amino acids. Examples include, but are not limited to, glycine, alanine, serine, threonine, cysteine, valine, leucine, isoleucine, methionine, proline, phenylalanine, tyrosine, and tryptophan.

In one aspect of the present invention, the carbohydrate or sugar is selected from the group consisting of mannitol, sorbitol, xylitol, maltitol, lactitol, sucrose, trehalose, mannose, maltose, lactose, xylose, ribose, glucose, raffinose, dextran, cyclodextrin, cellobiose, isomaltose, arabinose, glucosamine, and fructose. In a specific aspect of the present invention, the carbohydrate or sugar is selected from the group consisting of mannitol and trehalose.

In one aspect of the present invention, the stabilizer comprises at least one carbohydrate or sugar, each present at 0.01 to 10 w/v%. Specifically, the at least one carbohydrate or sugar included in the stabilizer may be present at 0.01 to 9.5 w/v%, 0.01 to 9 w/v%, 0.01 to 8.5 w/v%, 0.01 to 8 w/v%, 0.01 to 7.5 w/v%, 0.5 to 7.5 w/v%, or 1 to 7.5 w/v%. In a specific aspect of the present invention, the at least one carbohydrate or sugar included in the stabilizer is present at 1 to 6 w/v%, 1 to 5.9 w/v%, 1 to 5.8 w/v%, 1 to 5.7 w/v%, 1 to 5.6 w/v%, 1 to 5.5 w/v%, 1 to 5.4 w/v%, 1 to 5.3 w/v%, 1 to 5.2 w/v%, 1 to 5.1 w/v%, or 1 to 5 w/v%.

In a specific aspect of the present invention, the stabilizer may comprise two or more carbohydrates or sugars. Specifically, when two or more carbohydrates or sugars are included, one of the carbohydrates or sugars may be present at 1 to 3 w/v%, 1.1 to 3 w/v%, 1.2 to 3 w/v%, 1.3 to 3 w/v%, 1.4 to 3 w/v%, 1.5 to 3 w/v%, 1.5 to 2.9 w/v%, 1.5 to 2.8 w/v%, 1.5 to 2.7 w/v%, 1.5 to 2.6 w/v%, or 1.5 to 2.5 w/v%. Additionally, another carbohydrate or sugar may be present at 3 to 5 w/v%, 3.1 to 5 w/v%, 3.2 to 5 w/v%, 3.3 to 5 w/v%, 3.4 to 5 w/v%, 3.5 to 5 w/v%, 3.5 to 4.9 w/v%, 3.5 to 4.8 w/v%, 3.5 to 4.7 w/v%, 3.5 to 4.6 w/v%, or 3.5 to 4.5 w/v%. In a more specific aspect of the present invention, the stabilizer comprises two or more carbohydrates or sugars, wherein one of the carbohydrates or sugars is present at 1.5 to 2.5 w/v%, 1.55 to 2.5 w/v%, 1.6 to 2.5 w/v%, 1.65 to 2.5 w/v%, 1.7 to 2.5 w/v%, 1.75 to 2.5 w/v%, 1.75 to 2.45 w/v%, 1.75 to 2.4 w/v%, 1.75 to 2.35 w/v%, 1.75 to 2.3 w/v%, or 1.75 to 2.25 w/v%, and the other carbohydrate or sugar is present at 3.5 to 4.5 w/v%, 3.55 to 4.5 w/v%, 3.6 to 4.5 w/v%, 3.65 to 4.5 w/v%, 3.7 to 4.5 w/v%, 3.75 to 4.5 w/v%, 3.75 to 4.45 w/v%, 3.75 to 4.4 w/v%, 3.75 to 4.35 w/v%, 3.75 to 4.3 w/v%, or 3.75 to 4.25 w/v%.

In one aspect of the present invention, the carbohydrate or sugar comprises trehalose and mannitol. In one aspect of the present invention, the carbohydrate or sugar comprises trehalose and mannitol, each in an amount of 1 to 10 w/v%.

In a specific aspect of the present invention, the mannitol is present in an amount of 1 to 9 w/v%, 1 to 8 w/v%, 1 to 7 w/v%, 1 to 6 w/v%, 1 to 5 w/v%, 1.5 to 5 w/v%, 2 to 5 w/v%, 2.5 to 5 w/v%, 3 to 5 w/v%, 3.1 to 5 w/v%, 3.2 to 5 w/v%, 3.3 to 5 w/v%, 3.4 to 5 w/v%, 3.5 to 5 w/v%, 3.5 to 4.9 w/v%, 3.5 to 4.8 w/v%, 3.5 to 4.7 w/v%, 3.5 to 4.6 w/v%, or 3.5 to 4.5 w/v%. More specifically, the mannitol is present in an amount of 3.5 to 4.5 w/v%, 3.55 to 4.5 w/v%, 3.6 to 4.5 w/v%, 3.65 to 4.5 w/v%, 3.7 to 4.5 w/v%, 3.75 to 4.5 w/v%, 3.75 to 4.45 w/v%, 3.75 to 4.4 w/v%, 3.75 to 4.35 w/v%, 3.75 to 4.3 w/v%, or 3.75 to 4.25 w/v%.

In a specific aspect of the present invention, the trehalose is present in an amount of 1 to 3 w/v%, 1.1 to 3 w/v%, 1.2 to 3 w/v%, 1.3 to 3 w/v%, 1.4 to 3 w/v%, 1.5 to 3 w/v%, 1.5 to 2.9 w/v%, 1.5 to 2.8 w/v%, 1.5 to 2.7 w/v%, 1.5 to 2.6 w/v%, or 1.5 to 2.5 w/v%. More specifically, the trehalose is present in an amount of 1.5 to 2.5 w/v%, 1.55 to 2.5 w/v%, 1.6 to 2.5 w/v%, 1.65 to 2.5 w/v%, 1.7 to 2.5 w/v%, 1.75 to 2.5 w/v%, 1.75 to 2.45 w/v%, 1.75 to 2.4 w/v%, 1.75 to 2.35 w/v%, 1.75 to 2.3 w/v%, or 1.75 to 2.25 w/v%.

In one aspect of the present invention, the amino acid is selected from the group consisting of glycine, alanine, serine, threonine, cysteine, valine, leucine, isoleucine, methionine, proline, phenylalanine, tyrosine, tryptophan, aspartic acid, glutamic acid, asparagine, glutamine, histidine, lysine, and arginine. In one aspect of the present invention, the amino acid is glycine.

In one aspect of the present invention, the amino acid or a pharmaceutically acceptable salt thereof is present in an amount of 0.01 to 20 mg/ml. Specifically, the amino acid or a pharmaceutically acceptable salt thereof is present in an amount of 0.01 to 19 mg/ml, 0.01 to 18 mg/ml, 0.01 to 17 mg/ml, 0.01 to 16 mg/ml, 0.01 to 15 mg/ml, 0.01 to 14 mg/ml, 0.01 to 13 mg/ml, 0.01 to 12 mg/ml, 0.01 to 11 mg/ml, 0.01 to 10 mg/ml, 0.1 to 10 mg/ml, 0.2 to 10 mg/ml, 0.3 to 10 mg/ml, 0.4 to 10 mg/ml, 0.5 to 10 mg/ml, 0.6 to 10 mg/ml, 0.7 to 10 mg/ml, 0.8 to 10 mg/ml, 0.9 to 10 mg/ml, or 1 to 10 mg/ml. In a specific aspect of the present invention, the amino acid or a pharmaceutically acceptable salt thereof is present in an amount of 1 to 10 mg/ml, 1.5 to 10 mg/ml, 2 to 10 mg/ml, 2.5 to 10 mg/ml, 2.5 to 9.5 mg/ml, 2.5 to 9 mg/ml, 2.5 to 8.5 mg/ml, 2.5 to 8 mg/ml, or 2.5 to 7.5 mg/ml. In a more specific aspect of the present invention, the amino acid or a pharmaceutically acceptable salt thereof is present in an amount of 2.6 to 7.5 mg/ml, 2.7 to 7.5 mg/ml, 2.8 to 7.5 mg/ml, 2.9 to 7.5 mg/ml, 3 to 7.5 mg/ml, 3 to 7 mg/ml, 3 to 6.5 mg/ml, 3 to 6.4 mg/ml, 3 to 6.3 mg/ml, 3 to 6.2 mg/ml, 3 to 6.1 mg/ml, or 3 to 6 mg/ml.

In one aspect of the present invention, the stabilizer further comprises a non-ionic surfactant. The non-ionic surfactant may include, for example, poloxamers, polysorbates, and pluronics, but is not limited thereto.

In one aspect of the present invention, the stabilizer further comprises polysorbate. The polysorbate may be polysorbate 20, polysorbate 80, or a combination thereof.

In one aspect of the present invention, the stabilizer further comprises polysorbate 20, polysorbate 80, or a combination thereof in an amount of 0.001 to 0.05 w/v%.

In a specific aspect of the present invention, the polysorbate 20 or polysorbate 80 is present in an amount of 0.001 to 0.02 w/v%, 0.002 to 0.02 w/v%, 0.003 to 0.02 w/v%, 0.004 to 0.02 w/v%, 0.005 to 0.02 w/v%, 0.005 to 0.019 w/v%, 0.005 to 0.018 w/v%, 0.005 to 0.017 w/v%, 0.005 to 0.016 w/v%, or 0.005 to 0.015 w/v%. More specifically, the polysorbate 20 or 80 is present in an amount of 0.006 to 0.015 w/v%, 0.0065 to 0.015 w/v%, 0.007 to 0.015 w/v%, 0.0075 to 0.015 w/v%, 0.0075 to 0.014 w/v%, 0.006 to 0.013 w/v%, or 0.006 to 0.0125 w/v%.

In one aspect of the present invention, the pharmaceutical formulation has a potency of 60 to 130% relative to the reference potency. Specifically, the pharmaceutical composition has a potency of 70 to 120% relative to the reference potency.

In one aspect of the present invention, the pharmaceutical formulation has a purity (as measured by SEL-HPLC) of 90% or greater. Specifically, the purity is 91% or greater, 92% or greater, 93% or greater, or 94% or greater, and more specifically, the purity is 95% or greater.

In one aspect of the present invention, the pharmaceutical formulation is in a liquid or lyophilized form.

In a specific aspect of the present invention, the pharmaceutical formulation is in a lyophilized form. The pharmaceutical formulation of the present invention exhibits enhanced stability in the lyophilized form compared to the liquid form.

The pharmaceutical formulation of the present invention may take various forms depending on the specific product configuration, and may be provided in a filled form, such as in a tube, cartridge, syringe, polymer vial, or glass vial, but is not limited thereto. In addition, the present invention may be filled or otherwise processed by methods known to those of ordinary skill in the art.

In one aspect of the present invention, the present invention relates to a vial in which the pharmaceutical formulation is filled.

In one aspect of the present invention, the present invention relates to a syringe in which the pharmaceutical formulation is filled.

In one aspect of the present invention, the present invention may relate to a process of preparing a final pharmaceutical formulation-filled vial, wherein a bulk solution comprising a protein and an excipient is prepared in an amount of 1/2 by weight based on 1 part by weight of the final pharmaceutical formulation, and the prepared bulk solution is filled into a vial at twice the volume of the final pharmaceutical formulation. Specifically, 1 mL of a bulk solution comprising 0.5 to 10 mg/mL of recombinant stabilized Galectin-9 protein, 0.5 to 5 w/v% mannitol, 0.5 to 5 w/v% trehalose, 0.5 to 10 mg/mL of an amino acid, and 0.0005 to 0.025 w/v% polysorbate may be filled into a vial at 2 mL per vial and lyophilized to prepare a final formulation comprising 1 to 20 mg/mL of recombinant stabilized Galectin-9 protein, 1 to 10 w/v% mannitol, 1 to 10 w/v% trehalose, 1 to 20 mg/mL of an amino acid, and 0.001 to 0.05 w/v% polysorbate. More specifically, 1 mL of a bulk solution comprising 5 to 6 mg/mL of recombinant stabilized Galectin-9 protein, 2 w/v% mannitol, 1 w/v% trehalose, 2.5 mg/mL of an amino acid, and 0.005 w/v% polysorbate may be filled into a vial at 2 mL per vial and lyophilized to prepare a final formulation comprising 10 to 12 mg/mL of recombinant stabilized Galectin-9 protein, 4 w/v% mannitol, 2 w/v% trehalose, 5 mg/mL of an amino acid, and 0.01 w/v% polysorbate; however, the present invention is not limited thereto.

The present invention also relates to a pharmaceutical formulation for the prevention or treatment of a bone disease, comprising a therapeutically effective amount of the pharmaceutical formulation.

In the present invention, the bone disease may be at least one selected from the group consisting of osteoporosis, bone defects, osteomalacia, osteopenia, osteogenesis imperfecta, bone formation disorders, primary bone tumors, degenerative bone diseases, osteoarthritis, rheumatoid arthritis, bone fractures, osteonecrosis, periodontal disease, inflammatory alveolar bone resorption, inflammatory bone resorption, and Paget's disease, but is not limited thereto.

Furthermore, the present invention relates to a pharmaceutical formulation for the prevention of cancer or the treatment of autoimmune diseases, comprising a therapeutically effective amount of the pharmaceutical formulation.

In the present invention, the cancer may be one or more selected from the group consisting of colorectal cancer, lung cancer, thyroid cancer, gastric cancer, breast cancer, brain cancer, osteosarcoma, astrocytoma, melanoma, neuroglioma, glioblastoma, prostate cancer, ovarian cancer, cervical cancer, bladder cancer, renal cancer, gallbladder cancer, biliary tract cancer, esophageal cancer, colon cancer, rectal cancer, colorectal cancer, pancreatic cancer, hematologic cancer, leukemia, myelogenous leukemia, lymphoma, head and neck cancer, skin cancer, and liver cancer, but is not limited thereto.

In the present invention, the autoimmune disease may be one or more selected from the group consisting of systemic lupus erythematosus (SLE), insulin-dependent diabetes mellitus, multiple sclerosis, autoimmune encephalomyelitis, rheumatoid arthritis, juvenile rheumatoid arthritis, psoriatic arthritis, discoid lupus erythematosus, photosensitive dermatoses, autoimmune arthritis, myasthenia gravis, thyroiditis, experimental autoimmune uveitis, Hashimoto's thyroiditis, primary myxedema, thyrotoxicosis, pernicious anemia, autoimmune atrophic gastritis, Addison's disease, premature menopause, male infertility, juvenile diabetes, Goodpasture's syndrome, pemphigus vulgaris, pemphigus foliaceus, sympathetic ophthalmia, phacoanaphylactic uveitis, autoimmune hemolytic anemia, idiopathic leukopenia, primary biliary sclerosis, chronic active hepatitis (Hbs-negative), latent cirrhosis, ulcerative colitis, Sjögren's syndrome, scleroderma, Wegener's granulomatosis, polymyositis/dermatomyositis, and discoid LE, but is not limited thereto.

In addition, examples of the autoimmune disease include, but are not limited to, acute disseminated encephalomyelitis (ADEM), acute necrotizing hemorrhagic leukoencephalitis, Addison's disease, agammaglobulinemia, allergic asthma, allergic rhinitis, alopecia areata, amyloidosis, ankylosing spondylitis, antibody-mediated transplant rejection, anti-GBM/anti-TBM nephritis, antiphospholipid antibody syndrome (APS), autoimmune angioedema, autoimmune aplastic anemia, autoimmune autonomic neuropathy, autoimmune hepatitis, autoimmune hyperlipidemia, autoimmune immunodeficiency, autoimmune inner ear disease (AIED), autoimmune myocarditis, autoimmune pancreatitis, autoimmune diabetic retinopathy, autoimmune thrombocytopenic purpura (ATP), autoimmune thyroid disease, autoimmune urticaria, axonal and neuronal neuropathies, Balo disease, Behçet's disease, pemphigus foliaceus, cardiomyopathy, Castleman's disease, childhood lipodystrophy, Chagas disease, chronic fatigue syndrome, chronic inflammatory demyelinating polyneuropathy (CIDP), chronic recurrent multifocal osteomyelitis (CRMO), Churg-Strauss syndrome, cicatricial pemphigoid/benign mucosal pemphigoid, Crohn's disease, Cogan's syndrome, cold agglutinin disease, congenital heart block, coxsackie myocarditis, CREST syndrome, essential mixed cryoglobulinemia, demyelinating neuropathies, dermatitis herpetiformis, dermatomyositis, Devic's disease (neuromyelitis optica), discoid lupus erythematosus, Dressler's syndrome, endometriosis, eosinophilic fasciitis, erythema nodosum, experimental allergic encephalomyelitis, Evans syndrome, fibromyalgia, fibrosing alveolitis, giant cell arteritis (temporal arteritis), glomerulonephritis, Goodpasture's syndrome, granulomatosis with polyangiitis (GPA), Graves' disease, Guillain-Barré syndrome, Hashimoto's encephalopathy, Hashimoto's thyroiditis, hemolytic anemia, Henoch-Schönlein purpura, herpes gestationis, hypogammaglobulinemia, hypergammaglobulinemia, idiopathic thrombocytopenic purpura (ITP), IgA nephropathy, IgG4-related sclerosing disease, immunoregulatory lipoprotein abnormalities, inclusion body myositis, inflammatory bowel disease, insulin-dependent diabetes mellitus (type I), interstitial cystitis, juvenile arthritis, juvenile diabetes, Kawasaki syndrome, Eaton-Lambert syndrome, leukocytoclastic vasculitis, lichen planus, lichen sclerosus, ligneous conjunctivitis, linear IgA disease (LAD), lupus (SLE), Lyme disease, Meniere's disease, microscopic polyangiitis, mixed connective tissue disease (MCTD), monoclonal gammopathy of undetermined significance (MGUS), Mooren's ulcer, Mucha-Habermann disease, multiple sclerosis, myasthenia gravis, myositis, narcolepsy, neuromyelitis optica (Devic's disease), neutropenia, ocular cicatricial pemphigoid, optic neuritis, relapsing polychondritis, pediatric autoimmune neuropsychiatric disorders associated with streptococcal infections (PANDAS), paraneoplastic cerebellar degeneration, paroxysmal nocturnal hemoglobinuria (PNH), Parry-Romberg syndrome, Parsonage-Turner syndrome, pars planitis (intermediate uveitis), pemphigus, peripheral neuropathy, perivenous encephalomyelitis, pernicious anemia, POEMS syndrome, polyarteritis nodosa, autoimmune polyglandular syndrome type **I, II, or III,** polymyalgia rheumatica, polymyositis, post-myocardial infarction syndrome, post-pericardiotomy syndrome, progesterone dermatitis, primary biliary cirrhosis, primary sclerosing cholangitis, psoriasis, psoriatic arthritis, idiopathic pulmonary fibrosis, pyoderma gangrenosum, pure red cell aplasia, Raynaud's phenomenon, reflex sympathetic dystrophy, Reiter's syndrome, relapsing polychondritis, restless leg syndrome, retroperitoneal fibrosis, rheumatic fever, rheumatoid arthritis, sarcoidosis, Sheehan's syndrome, scleritis, scleroderma, Sjögren's syndrome, sperm and testis-related autoimmunity, stiff person syndrome, subacute bacterial endocarditis (SBE), Susac's syndrome, sympathetic ophthalmia, Takayasu's arteritis, temporal arteritis/giant cell arteritis, thrombotic thrombocytopenic purpura (TTP), Tolosa-Hunt syndrome, transverse myelitis, ulcerative colitis, undifferentiated connective tissue disease (UCTD), uveitis, vasculitis, vesiculobullous dermatoses, vitiligo, Waldenström's macroglobulinemia (WM), and Wegener's granulomatosis (granulomatosis with polyangiitis (GPA)).

Furthermore, the present invention relates to the use in the manufacture of a pharmaceutical formulation for the prevention or treatment of a bone disease.

Furthermore, the present invention relates to the use in the manufacture of a pharmaceutical formulation for the prevention or treatment of cancer or an autoimmune disease.

Furthermore, the present invention relates to the use of a pharmaceutical formulation for the prevention or treatment of a bone disease.

Furthermore, the present invention relates to the use of a pharmaceutical formulation for the prevention or treatment of cancer or an autoimmune disease.

Furthermore, the present invention relates to a method for treating a bone disease, comprising administering to a subject a therapeutically effective amount of a pharmaceutical formulation.

Furthermore, the present invention relates to a method for treating cancer or an autoimmune disease, comprising administering to a subject a therapeutically effective amount of a pharmaceutical formulation.

Furthermore, the present invention relates to the use of a pharmaceutical formulation for the prevention or treatment of a bone disease.

Furthermore, the present invention relates to the use of a pharmaceutical formulation for the prevention or treatment of cancer or an autoimmune disease.

Furthermore, the present invention relates to a method for treating a bone disease, the method comprising administering to a subject a therapeutically effective amount of a pharmaceutical formulation.

Furthermore, the present invention relates to a method for treating cancer or an autoimmune disease, the method comprising administering to a subject a therapeutically effective amount of a pharmaceutical formulation.

[MODE FOR CARRYING OUT THE INVENTION]

Hereinafter, the present invention will be described in further detail with reference to the following Examples and Experimental Examples.

It is to be understood, however, that the following Examples and Experimental Examples are provided only for illustrative purposes, and are not intended to limit the scope of the present invention.

**<Preparation Example** 1**> Production of Recombinant Stabilized Galectin-9 Protein (sGal-9)**

An expression vector comprising a gene encoding a recombinant stabilized Galectin-9 protein having the amino acid sequence of SEQ ID NO: 1 was constructed and introduced into *Escherichia coli* (E. coli) by a heat shock method. Expression of the recombinant protein was induced by culturing the E. *coli* in LB medium containing 50 μg/mL kanamycin, and adding arabinose when the optical density at 600 nm reached 0.7. Subsequently, the cells in which the expression of the recombinant protein had been induced were lysed and filtered. The target protein was then captured using cation exchange and affinity chromatography, thereby obtaining the recombinant stabilized Galectin-9 protein with high purity and high yield.

### <Preparation Example 2> Preparation of Stabilized Formulation

A formulation was prepared using the recombinant stabilized Galectin-9 protein (hereinafter referred to as "recombinant Galectin-9") obtained in Preparation Example 1. The composition of each formulation is as described in the following Experimental Examples. In one example, the purified solution was subjected to ultrafiltration (UF) and diafiltration (DF) using 5 mM histidine acetate (HisAce) buffer at pH 6.0 to adjust the protein concentration to 11-15 mg/mL. Thereafter, mannitol (2 w/v%), trehalose (1 w/v%), and glycine (2.5 mg/mL) were added and diluted to yield a final protein concentration of 5-7 mg/mL. The resulting solution was filtered through a 0.2 $\mu$m filter and filled into vials at 2.0 mL/vial, followed by lyophilization (freeze-drying).

### <Experimental Example 1> Evaluation Method for Buffer

### <Experimental Example 1-1> Appearance

The appearance of all samples was evaluated in accordance with the standard operating procedure (SOP) method PD-DPD-LAB-001-07. The evaluation included assessment of clarity, color, and visible particulates, which was performed using a YB-2 light box under both black and white backgrounds.

### <Experimental Example 1-2> pH

The pH was measured using a pH meter equipped with a glass electrode. The measurement was performed at least twice, and the average value was recorded.

### <Experimental Example 1-3> Protein Concentration

The protein concentration was measured using a Thermo UV spectrophotometer. The measurement was conducted in accordance with the SOP method (PD-DPD-EQU-088-01). For all evaluations, the absorbance coefficient was set to 1.0 AU $\times$ mL $\times$ mg$^{-1}$ $\times$ cm$^{-1}$, and each sample (2.5 $\mu$L) was measured at least twice. The average value was recorded.

### <Experimental Example 1-4> Size Exclusion Chromatography (SEC-HPLC)

Size exclusion chromatography (SEC-HPLC) was performed according to the following procedure.
If the sample concentration was greater than 1.0 mg/mL, the sample was diluted with mobile phase to 1.0 mg/mL prior to analysis. A 50 $\mu$g portion of the sample was injected into the HPLC system for analysis. SEC-HPLC analysis was conducted using a Hitachi Chromaster CM5000 system equipped with a UV detector (detection wavelength: 280 nm) and an Agilent TSKgel 2000SWxl column (7.8 mm $\times$ 300 mm, 5 $\mu$m). The column temperature was maintained at 25°C, and the loading volume was 50 $\mu$L. The mobile phase consisted of 300 mM sodium chloride, 10% isopropyl alcohol, and 50 mM phosphate buffer (pH 6.8 $\pm$ 0.1), with a flow rate of 0.4 mL/min.

### <Experimental Example 1-5> Protein Electrophoresis (SDS-PAGE)

SDS-PAGE analysis was performed using a heat block (DAIHAN

MaXtable™ 10H), an electrophoresis system (Bio-Rad BR 165-8033FC), and a rocker (Daihan RK-1D). The test samples were diluted at a ratio of 3:1 with 4$\times$ sample buffer and heated at 98°C for 5 minutes. A 12% acrylamide gel was prepared, and protein standards and test samples were loaded into separate wells. Electrophoresis was carried out at 100 V for 90 minutes. The gel with separated proteins was stained in CBB R-250 solution on a rocker for 30 minutes, followed by destaining in a destaining solution on a rocker for 30 minutes. After background destaining was complete, a distinct protein band was confirmed near 31 kDa for analysis.

### <Experimental Example 1-6> Potency Assay

## Cell Preparation

A 1 mL vial of Molt-4 T cells was thawed at 37°C for 1-2 minutes. The thawed cells were transferred into 10 mL of culture medium (RPMI1640 supplemented with 10% fetal bovine serum (FBS) and 1% penicillin/streptomycin), and centrifuged at 1,300 rpm for 3 minutes at room temperature (24°C). After removing the supernatant, the cell pellet was resuspended in fresh culture medium to a final concentration of $2\text{-}4 \times 10^5$ cells/mL. The cell suspension was transferred to a 25 cm$^2$ culture flask and incubated at 37°C in a 5% $CO_2$ incubator. When the cell density reached $1 \times 10^6$ cells/mL, the culture medium was replaced, and the cell concentration was adjusted to maintain $2\text{-}4 \times 10^5$ cells/mL. Subsequently, the doubling time and cell density were monitored and maintained accordingly.

## Potency Assay

The potency assay was conducted using Molt-4 T cells exhibiting a doubling time of 24 hours and a viability of 95% over a two-week period. The cells were diluted with fresh culture medium to a density of $5.6 \times 10^4$ cells/mL. Then, 90 $\mu$L of the cell suspension was dispensed into each well of a 96-well plate and incubated at 37°C in a 5% $CO_2$ incubator for 24 hours. The test sample was serially diluted in PBS(-) to concentrations of 300, 100, 30, 10, 3, 1, and 0 $\mu$g/mL. From each dilution, 10 $\mu$L was added to the wells such that the final concentrations in the medium were 30, 10, 3, 0.3, 0.1, and 0 $\mu$g/mL, respectively. After addition of the sample, the cells were incubated for an additional 24 hours at 37°C in a 5% $CO_2$ incubator. Following incubation, 10 $\mu$L of WST-1 detection solution was added to each well, and the plate was incubated for 2 hours at 37°C in a 5% $CO_2$ incubator. The optical density (OD) at 450 nm was then measured using a microplate reader. Changes in OD values and the $LD_{50}$ were calculated using a four-parameter logistic fit model.

### <Experimental Example 1-7> Osmolality

Osmolality was measured using an osmometer. The accuracy of the osmometer was verified before and after testing using a Clinitrol 290 mOsm/kg reference solution. Each sample was analyzed using a 20 $\mu$L aliquot.

### <Experimental Example 2> Liquid Formulation

### <Experimental Example 2-1> Comparative Analysis of Buffer Systems (1)

The stability of the sGal-9 protein was evaluated under stress conditions (37°C) using various pH/buffer systems. In each experiment, the concentration of sGal-9 was set at 2 mg/mL. The appearance of each sample was evaluated over a period of 4 weeks according to the appearance analysis described in Experimental Example 1. The buffer conditions used in the experiment are shown in Table 1 below.

[Table 1]

| Buffer | | | surfactant |
|---|---|---|---|
| F01 | 20 mM Histidine(His) | pH 6.0 | |
| F02 | 20 mM PBS(Phosphate buffer) | pH 7.4 | 0.02% PS80 (Polysorbate 80) |
| F03 | 20 mM acetate | pH 5.0 | |

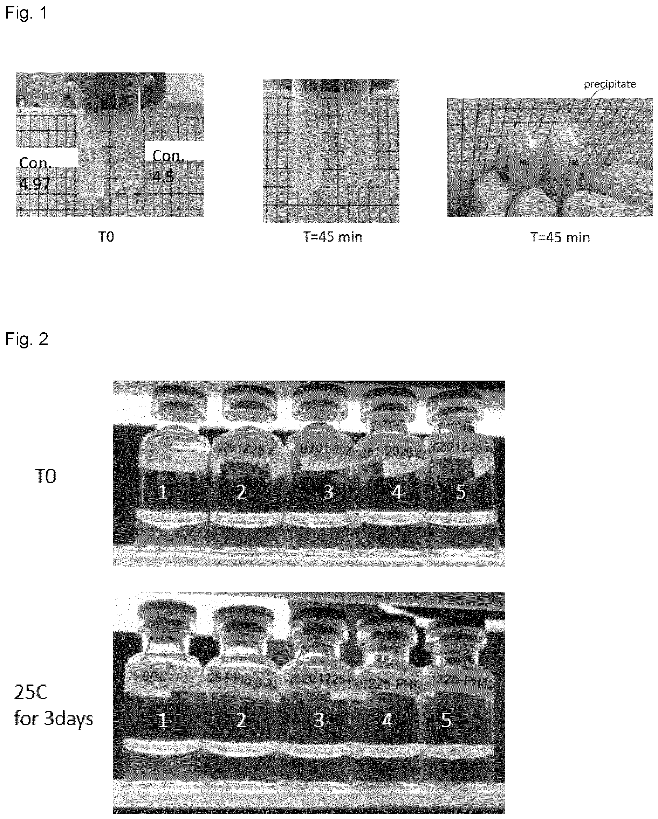

The experimental results are shown in FIGS. 1 and 2 and TABLE 2.
Fig. 1 shows a comparison between a histidine buffer and a PBS buffer. As shown in Fig. 1, precipitation was observed in the PBS buffer, confirming that the protein in the histidine buffer was more stable than in the PBS buffer.
TABLE 2 and Fig. 2 show a comparison between an acetate buffer and a PBS buffer. As shown in TABLE 2 and Fig. 2, a difference in particle number was observed when measured at 25°C three days after filtration with PBS, indicating that the acetate buffer exhibited superior performance compared to the PBS buffer.

[Table 2]

| Appearance | | |
|---|---|---|
| Identification | T0 | 25 °C / After 3 days |
| #1 | SO, P+ | SO, P+ |
| #2 | CL, P | CL, P |

(continued)

| Appearance | | |
|---|---|---|
| Identification | T0 | 25 °C / After 3 days |
| #3 | CL, P | CL, P |
| #4 | CL, PF | CL, P(2) |
| #5 | CL, PF | CL, P(>10) |
| (* CL(=clear liquid) / P(=particle) / P(n)(=particle number) / PF(= particle free)) (#1: 20 mM PBS, 50 mM NaCl, pH 7.3, 0.26 mg/mL; #2: in acetate buffer, pH 5.0, 0.62 mg/mL; #3: in acetate buffer, pH 5.3, 0.62 mg/mL; #4: #2 after filtration; #5: #3 after filtration) | | |

**<Experimental Example 2-2> Comparative Analysis of Buffer Systems (2) (Sodium Salts)**

The stability of the sGal-9 protein was evaluated using the same method as described in <Experimental Example 2-1>. The experiment was conducted over a period of two weeks and included assessments of appearance, UV absorbance, pH, SEC-HPLC, SDS-PAGE, and potency in accordance with the procedures described in Experimental Example 1. The buffer conditions used are shown in Table 3 below.

[Table 3]

| Buffer | | | surfactant |
|---|---|---|---|
| F01 | 20 mM Histidine Acetate (His Ace) | pH 6.0 | 0.04% PS80 (Polysorbate 80) |
| F02 | 20 mM Sodium Acetate (Na Ace) | pH 7.4 | |

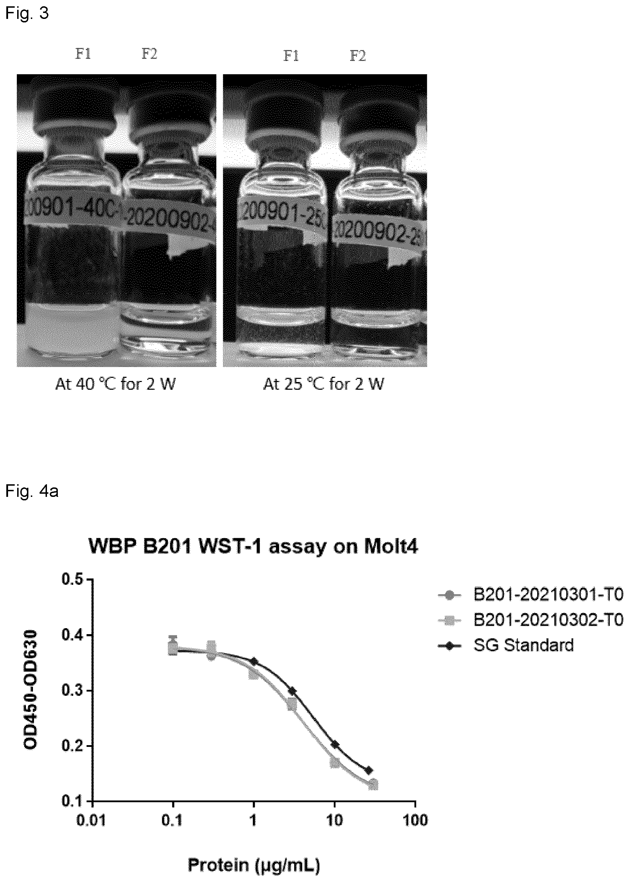

Appearance, UV absorbance, pH, SEC-HPLC, SDS-PAGE, and potency assays were performed at T0. At Week 1, appearance, UV absorbance, pH, SEC-HPLC, and SDS-PAGE analyses were conducted. At Week 2, appearance, UV absorbance, pH, SEC-HPLC, SDS-PAGE, and potency assays were again performed.
The results of the appearance analysis are shown in Table 4 and Fig. 3 below.

[Table 4]

| Identification | Appearance | | | | |
|---|---|---|---|---|---|
| | T0 | 40 °C | | 25 °C | |
| | | 1w | 2w | 1w | 2w |
| F01 | C, CL, P | O, P++ | O, P++ | C, SO, P+ | C, CL, P++ |
| F02 | C, CL, PF | SO, PF | SO, PF | C, CL, PF | C, CL, PF |
| (* C(=colorless) / SY(=slightly yellow)/ Y(=yellow)/ CL(=Clear liquid)/ SO(=slightly opalescent liquid)/ O(=opalescent liquid)/ FP(=free of visible particles) / PO(=particles observed, <5) / PO+(=a few visible particles)/ PO++(=many visible particles)) | | | | | |

The results of the SEC-HPLC analysis are shown in Tables 5 and 6.

[Table 5]

| Identification | Buffer | T0 | | | 25 °C, 1 w | | | 40 °C, 1w | | |
|---|---|---|---|---|---|---|---|---|---|---|
| | | Main | HMW | LMW | Main | HMW | LMW | Main | HMW | LMW |
| F1 | His Ace 6.0 | 89.5 | 10.5 | ND | 78.6 | 21.4 | ND | 42.4 | 44.3 | 13.3 |
| F2 | Na Acetate 5.0 | 73.0 | 27.0 | ND | 51.8 | 46.5 | ND | 0 | 75.2 | 24.8 |

[Table 6]

| Identification | Buffer | 25 °C, 2 w | | | 40 °C, 2w | | |
|---|---|---|---|---|---|---|---|
| | | Main | HMW | LMW | Main | HMW | LMW |
| F1 | His Ace 6.0 | 78.4 | 21.6 | ND | 0 | 76.8 | 23.2 |
| F2 | Na Acetate 5.0 | 43.4 | 54.7 | 1.9 | 0 | 68.8 | 31.2 |

The results of the potency assay are shown in Table 7.

[Table 7]

| Identif ication | Buffer | Concen tration (mg/mL ) | T0 | | | 40°C, 2 w | | | 25°C, 2w | | |
|---|---|---|---|---|---|---|---|---|---|---|---|
| | | | Potency($\mu$g/mL) | Max (OD 450-OD6 30) | Min (OD 450-OD6 30) | Potency($\mu$ g/mL) | Max (OD 450-OD6 30) | Min (OD 450-OD6 30) | Potency($\mu$g/mL) | Max (OD 450-OD6 30) | Min (OD 450-OD6 30) |
| F1 | His Ace 6.0 | 4.2 | 1.76 | 0.25 | 0.13 | NA | 0.27 | 0.27 | 2.32 | 0.26 | 0.14 |
| F2 | Na Ace tate 5.0 | 4.5 | 2.56 | 0.25 | 0.13 | NA | 0.27 | 0.27 | 6.46 | 0.27 | 0.14 |
| SG Standard | | 0.263 | 3.65 | 0.24 | 0.13 | 5.69 | 0.27 | 0.15 | 4.59 | 0.27 | 0.14 |

As shown in Table 4 and Fig. 3, histidine acetate exhibited whitish particles or a high number of visible particulates at both 25°C and 40°C, whereas no visible particulates were observed in the sodium acetate formulation. However, in terms of purity and potency, the protein in the histidine acetate buffer was found to be more stable than that in the sodium acetate buffer.

Furthermore, as shown in Tables 5 to 7, it was confirmed that the sodium salt form had a negative effect on the stability of the sGal-9 protein, indicating that the sodium salt form is unsuitable for use in the formulation.

### <Experimental Example 2-3> Comparative Analysis of Buffer Solutions (3) (pH/Excipient)

The stability of the sGal-9 protein was evaluated in the same manner as described in <Experimental Example 2-1>. The evaluation was conducted over a period of four weeks using the appearance, UV absorbance, pH, protein concentration (100 μL), SEC-HPLC, osmolality, and potency assay as described in Experimental Example 1. The buffer conditions are shown in Table 8 below.

[Table 8]

| Identification | Buffer | pH | Concentration | Sucrose | Glycine | PS (polysorbate 80) |
|---|---|---|---|---|---|---|
| | | | mg/mL | w/v% | mg/mL | w/v% |
| F01 | 20 mM Histidine Acetate (His Ace) | pH 5. 0 | 2 | 8 | 2 | 0.04 |
| F02 | 20 mM Histidine Acetate (His Ace) | pH 6. 0 | | | | |

At T0, tests were conducted for appearance, UV absorbance, pH, protein concentration (100 μL), SEC-HPLC, and osmolality. After one week, tests for UV absorbance, pH, protein concentration (100 μL), SEC-HPLC, and potency were performed at 40°C. After four weeks, tests for UV absorbance, pH, protein concentration (100 μL), SEC-HPLC, and potency were performed at 25°C.

The results of the appearance, pH, protein concentration, and osmolality tests are shown in Tables 9 and 10 below.

[Table 9]

| Identification | Buffer | pH | Concentration (mg/ml) | Appearance | | | Osmolality (mOsm/kg) |
|---|---|---|---|---|---|---|---|
| | | | | T0 | 40 °C | 25 °C | |
| | | | | | 1 w | 4 w | |
| F1 | 20 mM | 5.0 | 2.0 | C,CL,PF | C,CL,PF | C,CL,PF | 296 |
| F2 | His-Ace | 6.0 | | C,CL,PF | C,CL,PF | C,CL,PF | 297 |
| (* C(=colorless) / CL(=clear liquid) / PF(= particle free)) | | | | | | | |

[Table 10]

| Identification | Buffer | pH | Concentration (mg/ml) | pH | | | Concentration | | |
|---|---|---|---|---|---|---|---|---|---|
| | | | | T0 | 40 °C | 25 °C | T0 | 40 °C | 25 °C |
| | | | | | 1 w | 4 w | | 1w | 4 w |
| F1 | 20 mM | 5.0 | 2.0 | 5.0 | 5.0 | 5.0 | 2.0 | 2.1 | 2.0 |
| F2 | His-Ace | 6.0 | | 6.0 | 6.0 | 6.0 | 1.9 | 1.9 | 2.0 |

The results of the SEC-HPLC analysis are shown in Table 11.

[Table 11]

| Identification | Buffer | pH | Concentration (mg/ml) | T0 | | | 40 °C , 1W | | | 25 °C, 4W | | |
|---|---|---|---|---|---|---|---|---|---|---|---|---|
| | | | | %Monomer (Main)Peak | HMW | LMW | %Monomer (Main)Peak | HMW | LMW | %Monomer (Main)Peak | HMW | LMW |
| F1 | 20 mM His-Ace | 5. 0 | 2 | 95.2 | 4. 8 | N D | 3.2 | 95 .8 | 1. 1 | 69.2 | 30 .8 | N D |
| F2 | | 6. 0 | | 92.7 | 7. 3 | N D | 89.2 | 10 .8 | N D | 93.0 | 7. 0 | N D |

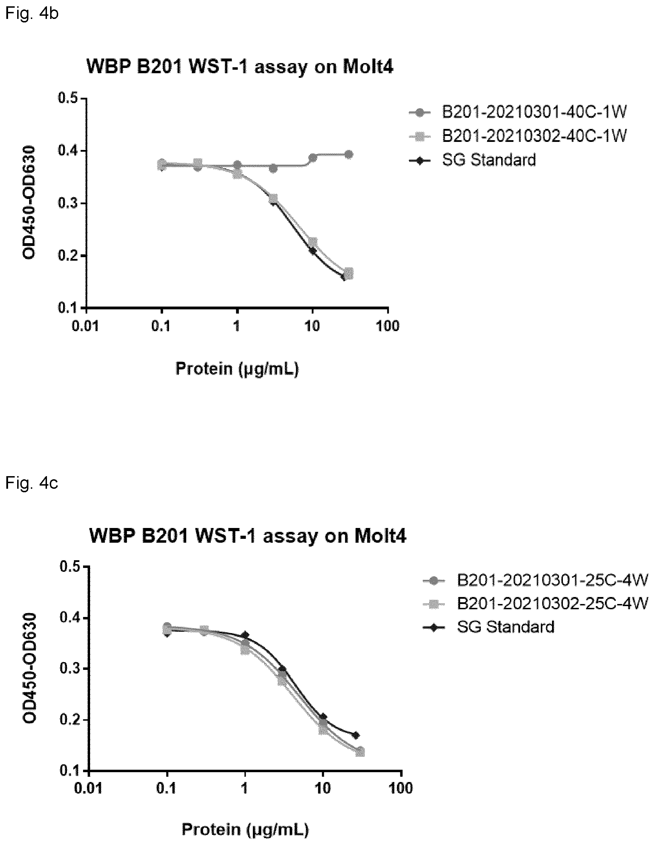

The results of the potency assay are shown in Tables 12 to 14 and FIGS 4a to 4c.

## (B201-20210301-T0 = F1 / B201-20210302-T0 = F2)

[Table 12]

| The result of the potency assay at T0. | | | | |
|---|---|---|---|---|
| Identification | Concentration (mg/ml) | Potency ( $\mu$g/mL) | Max (OD450-OD630) | Min (OD450-OD630) |
| F1 | 2.0 | 4.11 | 0.37 | 0.13 |
| F2 | 2.0 | 4.12 | 0.37 | 0.13 |
| SG Standard | 0.263 | 5.37 | 0.35 | 0.15 |

[Table 13]

| The result of the potency assay after 1 week at 40°C. | | | | |
|---|---|---|---|---|
| Identification | Concentration (mg/ml) | Potency ($\mu$g/mL) | Max (OD450-OD630) | Min (OD450-OD630) |
| F1 | 2.1 | NA | 0.37 | 0.39 |
| F2 | 2.0 | 6.63 | 0.37 | 0.16 |
| SG Standard | 0.263 | 5.44 | 0.37 | 0.16 |

[Table 14]

| The result of the potency assay after 4 weeks at 25°C. | | | | |
|---|---|---|---|---|
| Identification | Concentration (mg/ml) | **Potency** ($\mu$g/mL) | Max (OD450-OD630) | Min (OD450-OD630) |
| F1 | 2.0 | 4.88 | 0.37 | 0.14 |
| F2 | 2.0 | 4.12 | 0.37 | 0.14 |
| SG Standard | 0.263 | 4.37 | 0.36 | 0.17 |

From the above experimental results, it was confirmed that the histidine buffer (pH 6.0) exhibited superior performance compared to the histidine buffer (pH 5.0). In addition, it was confirmed that the sGal-9 protein exhibited stability when 8% sucrose, 0.04% polysorbate 80, and 2 mg/mL glycine were used as excipients.

### <Experimental Example 2-4> Comparative Analysis of Buffer Solutions (4) (pH)

The stability of the sGal-9 protein was evaluated in the same manner as described in <Experimental Example 2-1>. The experiment was conducted according to pH range and excipient conditions, and the buffer conditions are shown in Table 15 below.

[Table 15]

| Identification | Buffer | pH | Sucrose | Glycine | PS 20 (polysorbate 20) |
| --- | --- | --- | --- | --- | --- |
| | | | w/v% | mg/ml | w/v% |
| C1 | | 5.0 | 8 | - | - |
| C2 | | 6.0 | 8 | - | - |
| C3 | 20 mM His-Ace | 7.0 | 8 | - | - |
| C4 | | 6.0 | 8 | - | 0.02 |
| C5 | | 6.0 | 8 | 2 | 0.02 |

The results of the SEC-HPLC analysis, protein concentration, and potency assay are shown in Table 16 below.

[Table 16]

| | | C1 | C2 | C3 | C4 | C5 |
| --- | --- | --- | --- | --- | --- | --- |
| T0 | Purity: SEC-HPLC Area(%) | 92.5 | 92.2 | 89.6 | 85.4 | 85.4 |
| | Protein concentration (A280 / BF) | 2.0 / 2.01 | 2.1 / 1.78 | 2.1 / 1.76 | 2.2 / 2.24 | 2.2 / 2.3 |
| | Potency (ug/ml) | 6.39 | | | 5.03 | |
| T4D | Purity: SEC-HPLC Area(%) | 35.44 / | 97.1 | 66.83 | 90.17 | 90.14 |
| | Protein concentration (A280) | 2.3 / 2.09 | 2.2 / 1.8 | 2.9 / 1.96 | 2.4 / 1.96 | 2.6 / 1.82 |
| | Potency (ug/ml) | > 31.5 | 4.56 | 5.90 | 3.26 | 5.51 |

From the above experimental results, it was confirmed that the histidine acetate buffer at pH 6.0 exhibited superior performance compared to buffers at other pH values. In addition, it was confirmed that the effects of other excipients, such as polysorbate 20 (PS 20) or glycine, were not clearly observed.

**<Experimental Example 2-5> Comparative Analysis of Buffer Solutions (5) (Concentration)**

The stability of the sGal-9 protein was evaluated in the same manner as described in <Experimental Example 2-1>. The experiment was conducted based on concentration variations, and the buffer conditions are shown in Table 17 below.

[Table 17]

| Identification | Buffer | Mannitol | Sucroese | PS 20 (polysorbate 20) | Glycine |
| --- | --- | --- | --- | --- | --- |
| | | (w/v %) | (w/v %) | (w/v %) | (mg/ml) |
| C1 | | - | 8 | - | 5 |
| C2 | 20mM His-Ace pH6.0 | - | 8 | 0.02 | 5 |
| C3 | | 5 | - | - | 5 |
| C4 | | - | 8 | - | 5 |
| C5 | 10mM His-Ace pH6.0 | - | 8 | 0.02 | 5 |
| C6 | | - | 8 | - | 5 |
| C7 | 30mM His-Ace pH6.0 | - | 8 | 0.02 | 5 |
| C8 | | 5 | - | - | 5 |

The results of the SEC-HPLC analysis, protein concentration, and potency assay are shown in Table 18 below.

[Table 18]

| Identification | | 20mM His-Ace / 5mg/ml Glycine | | | 10mM His-Ace / 5mg/ml Glycine | | 30mM His-Ace / 5mg/ml Glycine | | |
|---|---|---|---|---|---|---|---|---|---|
| | | 8% Sucrose | 8% Sucrose 0.02 % PS2 0 | 5% Mannitol | 8% Sucrose | 8% Sucrose 0.02 % PS2 0 | 8% Sucrose | 8% Sucrose 0.02 % PS2 0 | 5% Mannitol |
| | | C1 | C2 | C3 | C4 | C5 | C6 | C7 | C8 |
| T0 | Purity (%) | 96.74 | 86.79 | 96.41 | 96.17 | 95.55 | 96.13 | 96.70 | 91.73 |
| | A280 / BF (mg/ ml) | 2.52 / 2.45 | 2.44 / 2.42 | 1.84 / 1.84 | 1.80 / 1.85 | 1.77 / 1.91 | 2.46 / 2.34 | 2.45 / 2.35 | 1.79 / 1.89 |
| | Poten cy (ug/m l) | 0.94 | 0.88 7 | 1.76 5 | 0.88 4 | 0.59 8 | 0.70 3 | 0.99 8 | 0.80 3 |
| T2 W | Purity (%) | 97.3 0 | 96.2 3 | 96.3 7 | 97.7 9 | 96.7 1 | 95.6 0 | 92.1 9 | 94.2 5 |
| | A280 / BF (mg/ ml) | 2.98 / 2.28 | 3.30 / 2.16 | 2.15 / 1.68 | 2.01 / 1.47 | 2.21 / 1.53 | 3.00 / 2.32 | 3.75 / 1.88 | 2.47 / 1.35 |
| | Poten cy (ug/m l) | 3.00 | 4.34 | 3.71 | 2.74 | 3.94 | 2.98 | 5.88 | 4.12 |
| T3 W | Purity (%) | 95.8 0 | 93.5 3 | 95.0 8 | 97.9 1 | 97.0 8 | 94.1 0 | 88.6 9 | 95.7 0 |
| | A280 / BF (mg/ ml) | 3.26 / 1.79 | 3.93 / 1.69 | 2.57 / 1.18 | 2.13 / 1.34 | 2.46 / 1.26 | 3.42 / 1.65 | 4.34 / 1.42 | 3.08 / 0.97 |
| | Poten cy (ug/m l) | 3.17 2 ± 0.47 1 | 5.77 8 ± 0.45 5 | 3.32 8 ± 0.1 89 | 1.69 6 ± 0.3 61 | 1.91 9 ± 0.2 64 | 1.92 1 ± 0.3 10 | 3.96 5 ± 1.2 58 | 0.58 7 ± 0.24 9 |

From the above experimental results, it was confirmed that the histidine acetate buffer exhibited greater protein stability at concentrations of 10 mM and 20 mM compared to 30 mM, in the order of 10 mM > 20 mM > 30 mM, indicating enhanced stability at lower concentrations.

**<Experimental Example 2-6> Comparative Analysis of Buffer Solutions (6)**

The stability of the sGal-9 protein was evaluated in the same manner as described in <Experimental Example 2-1>. The buffer conditions are shown in Table 19 below.

[Table 19]

| Identification | Buffer | Mannitol (w/v %) | Sucrose (w/v %) | PS 20 (polysorbate 20) (w/v %) | Glycine (mg/ml) |
|---|---|---|---|---|---|
| C1 | 20mM His-Ace pH6.0 | - | 8 | - | 5 |
| C2 | | - | 8 | 0.02 | 5 |
| C3 | | 5 | - | - | 5 |
| C4 | 10mM His-Ace pH6.0 | - | 8 | - | 5 |
| C5 | | - | 8 | 0.02 | 5 |

The results of the SEC-HPLC analysis, protein concentration, and potency assay are shown in Table 20 below.

[Table 20]

| Identification | | 20mM His-Ace / 5mg/ml Glycine | | | 10mM His-Ace / 5mg/ml Glycine | |
|---|---|---|---|---|---|---|
| | | 8% Sucrose | 8% Sucrose 0.02% PS20 | 5% Mannitol | 8% Sucrose | 8% Sucrose 0.02% PS20 |
| | | C1 | C2 | C3 | C4 | C5 |
| T0 | Purity(%) | 98.16 | 98.06 / | 97.17 | 99.35 | 97.90 |
| | A280 / BF (mg/ml) | 2.04 / 1.95 | 2.05 / 2.24 | 2.06 / 1.80 | 1.93 / 1.97 | 2.07/ 2.34 |
| | Potency (ug/ml) | 7.642 ± 1.873 | 2.447 ± 0.559 | 3.113 ± 0.394 | 2.630 ± 0.473 | 1.248 ± 0.113 |
| T2W | Purity(%) | 91.84 | 85.76 | 90.24 | 92.83 | 80.63 |
| | A280 / BF (mg/ml) | 2.49 / 2.07 | 3.53 / 1.67 | 2.53 / 1.64 | 2.23/ 1.60 | 2.82/ 1.61 |
| | Potency (ug/ml) | 2.454 ± 0.455 | 5.551 ± 3.583 | 4.771 ± 1.287 | 5.084 ± 1.298 | 5.834 ± 1.624 |

From the above experimental results, it was confirmed that the histidine acetate buffer was most effective at 10 mM, and that greater stability was observed when sucrose was used.

<Experimental Example 3> Lyophilized Formulation

Based on the results obtained from Experimental Example 2, the buffer and pH were maintained at 10 mM histidine acetate (pH 6.0), and excipients and formulations were compared. The lyophilized formulation was prepared by formulating the sGal-9 protein with each excipient, filling the formulation into 10 mL vials to contain 10 mg of sGal-9 per vial, and then subjecting it to lyophilization. Analyses were performed by reconstituting the lyophilized samples in 1.0 mL of deionized water at each time point.

<Experimental Example 3-1> Comparative Analysis of Excipients (1)

The stability of the sGal-9 protein was evaluated in the same manner as described in <Experimental Example 2-1>. The excipient conditions are shown in Table 21 below.

[Table 21]

| Identification | Buffer | pH | Sucrose (w/v %) | PS 80 (polysorbate 80) (w/v %) | Glycine (mg/ml) |
|---|---|---|---|---|---|
| C1 | 10 mM | 6.0 | 8 | - | 5 |
| C2 | His-Ace | 6.0 | 8 | 0.01 | 5 |

The results of the SEC-HPLC analysis, protein concentration, and potency assay are shown in Table 22 below.

[Table 22]

| Identification | | 10mM His-Ace pH6.0 / 8% Sucrose, 5mg/ml Glycine | | 10mM His-Ace pH6.0 / 8% Sucrose, 5mg/ml Glycine / 0.01% PS80 | |
|---|---|---|---|---|---|
| | | 25 °C | 37 °C | 25 °C | 37 °C |
| Befor e FD | Purity(% ) | 97.94 | | 98.23 | |
| | A280 (mg/ml) | 2.26 (11.3mg) | | 2.30 (11.5mg) | |

(continued)

| Identification | | 10mM His-Ace pH6.0 / 8% Sucrose, 5mg/ml Glycine | | 10mM His-Ace pH6.0 / 8% Sucrose, 5mg/ml Glycine / 0.01% PS80 | |
|---|---|---|---|---|---|
| | | 25 °C | 37 °C | 25 °C | 37 °C |
| T0 (After FD) | Purity (%) | 97.07 | | 96.29 | |
| | A280 (mg/ml) | 8.52 | | 9.42 | |
| | Potency (ug/ml) | 0.78±0.16 | | 1.76±0.15 | |
| T2W (37°C) T3W (25°C) | | T3W(25°C) | T2W(37°C) | T3W(25°C) | T2W(37°C) |
| | Purity (%) | 95.71 | 92.87 | 96.00 | 71.98 |
| | A280 (mg/ml) | 8.26 | 8.90 | 9.05 | 9.46 |
| | Potency (ug/ml) | | 1.358±0.136 | | 1.062±0.028 (Remarks - 1.124, 1.346 ±0.217) |

From the above experimental results, it was confirmed that the purity decreased under stressed conditions (37°C) at the 2-week (T2W) analysis point. In addition, it was confirmed that the inclusion of PS80 (0.01%) resulted in reduced stability.

**<Experimental Example 3-2> Comparative Analysis of Excipients** (2)

The stability of the sGal-9 protein was evaluated in the same manner as described in <Experimental Example 2-1>. The excipient conditions are shown in Table 23 below.

[Table 23]

| Identification | Buffer | Sucrose (w/v %) | Mannitol (w/v %) | Trehalose (w/v %) | Glycine (mg/ml) | PS 80 (w/v %) |
|---|---|---|---|---|---|---|
| C1 | 10 mM | 8 | - | - | 5 | 0.01 |
| C2 | His-Ace | - | 5 | - | 5 | 0.01 |
| C3 | (pH 6.0) | - | 4 | 2 | 5 | 0.01 |
| C4 | | 2 | 4 | - | 5 | 0.01 |

The results of the SEC-HPLC analysis, protein concentration, and potency assay are shown in Tables 24 and 25 below.

[Table 24]

| Identification | | C1 | C2 | C3 | C4 | STD |
|---|---|---|---|---|---|---|
| Before FD | Purity (%) | 96.81 | 99.47 | 96.43 | 96.82 | 97.19 |
| | A280 (mg/ml) | 3.23 | 3.21 | 3.21 | 3.26 | 1.85 |
| T0 | Purity (%) | 97.82 | 97.63 | 97.82 | 97.71 | 96.53 |
| | A280 (mg/ml) | 9.55 | 9.72 | 9.82 | 9.69 | |
| T0 (5°C, 1d) | Purity (%) | 97.52 | 97.4 | 97.54 | 97.56 | 97.69 |
| | A280 (mg/ml) | 9.41 | 9.54 | 9.83 | 9.82 | |

[Table 25]

|  |  | C1 | | C2 | | C3 | | C4 | | ST D |
| --- | --- | --- | --- | --- | --- | --- | --- | --- | --- | --- |
|  |  | Room tempe rature | 37 °C | Room tempe rature | 37 °C | Room tempe rature | 37 °C | Room tempe rature | 37 °C | |
| Before FD | Purity (%) | 96.81 | | 99.47 | | 96.43 | | 96.82 | | 97.1 9 |
| | A280 (mg/ ml) | 3.23 | | 3.21 | | 3.21 | | 3.26 | | 1.8 5 |
| T0 | Purity (Area %) | 97.82 | | 97.63 | | 97.82 | | 97.71 | | 96. 53 |
| | A280 (mg/ ml) | 9.55 | | 9.72 | | 9.82 | | 9.69 | | |
| T1 W | Purity (%) | 96.9 | | 97.65 | | 97.57 | | 7.67 | | 96. 78 |
| | A280 (mg/ ml) | 9.54 | | 9.75 | | 9.70 | | 9.66 | | |
| T2 W (25 °C) | Purity (%) | 98.09 | | 97.94 | | 98.03 | | 97.71 | | 97. 06 |
| | A280 (mg/ ml) | 9.74 | | 9.65 | | 9.91 | | 9.90 | | |

From the above experimental results, it was confirmed that no changes were observed in each experimental parameter (SEC-HPLC, UV, and PAGE) over 24 hours. In addition, it was confirmed that the formulation containing mannitol exhibited stability.

**<Experimental Example 3-3> Comparative Analysis of Excipients (3)**

The stability of the sGal-9 protein was evaluated in the same manner as described in <Experimental Example 2-1>. The excipient conditions are shown in Table 26 below.

[Table 26]

| Identification | Buffer | Mannitol | Trehalose | Glycine | PS80 |
| --- | --- | --- | --- | --- | --- |
| | | (w/v %) | (w/v %) | (mg/ml) | (w/v %) |
| C1 | 10mM HisAce pH6.0 | 5 | - | 5 | - |
| C2 | | 4 | 2 | 5 | 0.01 |
| C3 | | 4 | 2 | 5 | - |
| C4 | | 5 | 5 | - | - |

The results of the SEC-HPLC analysis, protein concentration, and potency assay are shown in Tables 27 and 28 below.

[Table 27]

| Identification | | C1 | C2 | C3 | C4 | STD |
| --- | --- | --- | --- | --- | --- | --- |
| Before FD | Purity (%) | 97.48 | 95.95 | 98.20 | 98.21 | 97.36 |
| | A280 (mg/ ml) | 3.23 | 3.21 | 3.21 | 3.26 | |
| | Pote ncy (ug/m l) | 1.91 ± 0.1 8 | 2.05 ± 0.0 4 | 2.16 ± 0.1 43 | 1.860 ± 0. 29 | 1.59 ± 0. 45 |
| T0 | Purity (%) | 97.10 | 96.66 | 96.49 | 96.82 | |
| | A280 (mg/ ml) | 10.09 | 9.85 | 9.88 | 9.27 | |
| | Pote ncy (ug/m l) | 2.274 ± 0. 300 | 2.812 ± 0. 421 | 2.446 ± 0. 216 | 3.366 ± 0. 448 | 3.059 ± 0.46 |

[Table 28]

| | | C1 | C2 | C3 | C4 | STD |
|---|---|---|---|---|---|---|
| T4W Room Temperature (25°C) | Purity (%) | 96.20 | 95.97 | 95.63 | 96.05 | |
| | A280 (mg/ ml) | 1.542 | 1.445 | 1.367 | 1.389 | |
| | Potency (ug/ ml) | 3.286± 0.324 | 2.450± 0.181 | 2.447± 0.714 | 3.241± 0.234 | 3.059± 0.46 |
| T4W (37°C) | Purity (%) | 94.14 | 96.24 | 95.63 | 96.63 | |
| | A280 (mg/ ml) | 1.533 | 1.427 | 1.380 | 1.308 | |
| | Potency | 3.792± 0.29 | 3.063± 0.07 | 3.451± 0.33 | 2.42±0. 26 | 2.37±0 .29 |
| | (ug/ ml) | (63%/st d.) | (77%/st d.) | (69%/st d.) | (98%/st d.) | |

From the above experimental results, it was confirmed that the formulation exhibited stability when trehalose was included.

**<Experimental Example 3-4> Comparative Analysis of Excipients (4)**

The stability of the sGal-9 protein was evaluated in the same manner as described in <Experimental Example 2-1>. The excipient conditions are shown in Table 29 below.

[Table 29]

| Identification | Buffer | Mannitol (w/v %) | Trehalose (w/v %) | Glycine (mg/ml) | PS80 (w/v %) | Dithiothreitol (DTT) (mM) |
|---|---|---|---|---|---|---|
| C1 | 10mM HisAce pH6.0 | 4 | 2 | 5 | - | |
| C2 | | 4 | 2 | 5 | 0.01 | |
| C3 | | 4 | 2 | 5 | 0.01 | 1.0 |
| C4 | | 4 | 2 | 5 | - | 1.0 |
| C5 | | 2 | 4 | 5 | - | - |

The results of the SEC-HPLC analysis, protein concentration, and potency assay are shown in Tables 30 to 32 below.

[Table 30]

| | | C1 | C2 | C3 | C4 | C5 | STD |
|---|---|---|---|---|---|---|---|
| Before FD | Purity (%) | 97.59 | 96.92 | 96.44 | 96.94 | 97.40 | 95.91 |
| | A280 (mg/ ml) | 1.518 | 1.718 | 1.725 | 1.53 | 1.587 | |
| | Potency (ug/ ml) | 1.689 ± 0.87 | 2.476 ± 1.133 | 2.773 ± 0.146 | 1.515 ± 5.469 | 2.134 ± 0.15 | 3.963 |
| After FD (T0) | Purity (%) | 97.08 | 97.14 | 96.28 | 96.37 | 96.72 | 96.46 |
| | A280 (mg/ ml) | 1.296 | 1.442 | 1.418 | 1.353 | 1.347 | |
| | Potency (ug/ ml) | 2.862 ± 0.09 | 2.967 ± 0.19 | 2.624 ± 0.11 | 1.921 ± 0.04 | 3.244 ± 0.19 | 2.172 ± 0.06 3.077± 0.22 |

[Table 31]

|  |  | C1 | C2 | C3 | C4 | C5 | STD |
|---|---|---|---|---|---|---|---|
| Room temperature (25°C) | Purity (%) | 96.46 | 96.78 | 96.31 | 96.42 | 96.49 | 95.6 3 |
|  | A280 (mg/ ml) | 1.37 | 1.48 | 1.49 | 1.38 | 1.38 |  |
|  | Potency (ug/m l) | 2.06 ± 0 .27 | 2.60 ± 0 .47 | 2.75 ± 1 .39 | 2.23 ± 0 .58 | 1.74 ± 0 .36 | 2.4 7 |
| 37°C | Purity (%) | 96.43 | 96.64 | 96.14 | 95.74 | 96.28 |  |
|  | A280 (mg/ ml) | 1.37 | 1.47 | 1.47 | 1.34 | 1.30 |  |
|  | Potency (ug/m l) | 5.84 ± 0 .71 | 6.85 ± 0 .90 | 6.02 ± 1 .18 | 5.01 ± 1 .39 | 2.17 ± 0 .58 | 5.6 0 |

[Table 32]

|  |  | C1 | C2 | C3 | C4 | C5 | STD |
|---|---|---|---|---|---|---|---|
| Low Temperature (5°C) | Purity (%) | 96.75 | 96.8 | 96.02 | 96.37 | 96.75 |  |
|  | A28 0 (mg/ ml) | 9.279 | 10.111 | 10.122 | 9.415 | 9.372 | 1.8 |
|  | Pote ncy (ug/ ml) | 5.261 ±2.2 | 3.358 ±3.19 | 1.762 ±0.37 | 2.227 ±0.86 | 2.789 ±1.2 | 1.621 ±1.16 |
| Room Temperature (25°C) | Purity (%) | 96.44 | 96.75 | 96.16 | 96.44 | 96.68 |  |
|  | A28 0 (mg/ ml) | 9.284 | 9.362 | 10.227 | 10.241 | 9.423 | 1.8 |
|  | Pote ncy (ug/ ml) | 2.911 ±0.04 | 2.732 ±0.17 | 2.897 ±0.27 | 3.746 ±1.3 | 2.789 ±1.2 | 2.106 ±0.35 |
| 37°C | Purit y (%) | 95.07 | 96.27 | 95.7 | 95.56 | 95.91 |  |
|  | A28 0 (mg/ ml) | 9.29 | 9.97 | 10.11 | 9.34 | 9.32 | 1.8 |
|  | Pote ncy (ug/ ml) | 4.337 ±1.33 | 5.573 ±1.12 | 4.258 ±1.83 | 2.978 ±1.77 | 4.218 ±1.04 | 1.484 ±0.25 |

From the above experimental results, it was confirmed that there were no significant differences in stability among the candidate excipients.

**<Experimental Example 3-5> Comparative Analysis of Excipients (5)**

The stability of the sGal-9 protein was evaluated in the same manner as described in <Experimental Example 2-1>. The excipient conditions are shown in Table 33 below.

[Table 33]

| Identification | Buffer | Mannitol (w/v %) | Trehalose (w/v %) | Glycine (mg/ml) | PS80 (w/v %) |
|---|---|---|---|---|---|
| C1 | 10mM HisAce pH6.0 | 4 | 2 | 5 | - |
| C2 | | 4 | 2 | 5 | 0.01 |

The results of the SEC-HPLC analysis, protein concentration, and potency assay are shown in Tables 34 to 37 below.

[Table 34]

|  |  | C1 | C2 | STD |
|---|---|---|---|---|
| Before FD | Purity (%) | 96.48 | 96.60 | 89.33 |
|  | A280 (mg/ml) | 11.0 | 11.4 | 1.9 |
|  | Potency (ug/ml) | 1.94±0.15 | 2.287±0.506 | 1.848±0.255 |

(continued)

| | | C1 | C2 | STD |
|---|---|---|---|---|
| After FD (T0) | Purity (%) | 95.57 | 96.16 | |
| | A280 (mg/ml) | 10.8 | 10.6 | |
| | Potency (ug/ml) | 1.764±0.173 | 1.142±0.381 | |

[Table 35]

| | | C1 | C2 | STD (1.0mg/ml) |
|---|---|---|---|---|
| T1M (25°C) | Purity (%) | 97.4 | 98.08 | 97.43 |
| | A280 (mg/ml) | 10.79 | 10.86 | 1.0 |
| | Potency (ug/ml) | 2.179±0.181 (Std x 127.7%) | 3.015±0.041 (std x 92.27%) | 2.782±0.412, 1.723±0.117 |
| T1M (37°C) | Purity (%) | 97.88 | 97.51 | 97.43 |
| | A280 (mg/ml) | 10.88 | 11.13 | 1.0 |
| | Potency (ug/ml) | 1.324±0.127 (std x 120.4%) | 1.858±0.142 (std. x 96.5%) | |

[Table 36]

| | | C1 | C2 | STD (1.8mg/ml) |
|---|---|---|---|---|
| T2M (5°C) | Purity (%) | 97.32 | 97.52 | 98.72 |
| | A280 (mg/ml) | 10.82 | 10.92 | 1.8 |
| | Potency (ug/ml) | 2.982±1.34 (74.08%) | 2.229±0.62 (99.1%) | 2.209±0.21 2.762±0.35 |
| T2M (25°C) | Purity (%) | 96.6 | 96.51 | 98.72 |
| | A280 (mg/ml) | 10.88 | 10.94 | |
| | Potency (ug/ml) | 2.766±0.93 (99.86%) | 3.048±0.78 (90.62%) | |
| T2M (37°C) | Purity (%) | 97.07 | 96.67 | |
| | A280 | 10.86 | 10.83 | |
| | (mg/ml) | | | |
| | Potency (ug/ml) | 3.641±2.66 (75.8%) | 3.609±0.37 (76.5%) | |

[Table 37]

| | | C1 | C2 | STD (0.9mg/ml) |
|---|---|---|---|---|
| T3M (25°C) | Purity (%) | 97.89 | 97.57 | |
| | A280 (mg/ml) | 10.75 | 10.59 | |
| | Potency (ug/ml) | 1.512±0.41 (stdx157.7%) | 1.776±0.15 (stdx134.3%) | 2.385±0.26 |
| T3M (37°C) | Purity (%) | 97.57 | 96.83 | |
| | A280 (mg/ml) | 10.64 | 10.48 | |
| | Potency (ug/ml) | 2.219±0.08 (stdx107.5%) | 1.871±0.44 (stdx127.5%) | |

From the above experimental results, it was confirmed that the presence or absence of PS80 did not result in any significant difference in stability.

**<Experimental Example 3-6> Comparative Analysis of Excipients (6)**

The lyophilized formulation was prepared in the same manner as described in <Preparation Examples 2 and 3>.

The excipient conditions are shown in Table 38 below.

[Table 38]

| Identification | Buffer | Mannitol (w/v %) | Trehalose (w/v %) | Glycine (mg/ml) | PS80 (w/v %) |
|---|---|---|---|---|---|
| C1 | 10mM HisAce pH6.0 | 4 | 2 | 5 | 0.01 |
| C2 | | 2 | 2 | 5 | 0.01 |

In addition, the stability of the sGal-9 protein was evaluated in the same manner as described in <Experimental Example 2-1>. The purpose of the analysis, which included SEC-HPLC, IEX-HPLC, SDS-PAGE, protein concentration, and potency assays, was to compare the stability of the sGal-9 protein between 2% mannitol and 4% mannitol formulations. The test results are shown in Tables 39 and 40 below.

[Table 39]

| Analysis items | | SEC Area % | IEX Area % | A280 (mg/ml) | Potency (ug/ml) |
|---|---|---|---|---|---|
| Before FD | C1 | 97.73 | 96.88 | 10.78 | 1.277±0.102(87.16%), 1.075 ±0.038(96.59%) |
| | C2 | 97.83 | 97.04 | 10.73 | 1.26±0.051(113.2%), 0.982±0.076(88.23%) |
| After FD (10/7) | C1 | 97.47 | 96.90 | 10.56 (97.96%) | 0.984±0.051(89.05%), 0.992 ±0.068(89.77%) |
| | C2 | 96.87 | 96.88 | 9.95 (92.73%) | 1.195±0.064(108.1%), 1.165 ±0.018(104.5%) |
| Wstd | B8 (1mg/ml) | 97.73 | 96.07 | | 1.113±0.034 1.105±0.096 |

[Table 40]

| Analysis items | | SEC Area % | IEX Area % | A280 (mg/ml) | Potency (ug/ml) |
|---|---|---|---|---|---|
| 37°C T1M (11/7) | C1 | 97.10 | 99.80 | 10.26 | 1.547±0.192 (std x 117.1%) |
| | C2 | 97.23 | 99.83 | 9.89 | 1.365±0.027 (std x 132.7%) |
| 37°C T2M (12/7) | C1 | 97.21 | 98.65 | 10.2 (96.59%) | 1.845±0.013 (std x 72.9%) |
| | C2 | 97.44 | 98.87 | 9.7 (97.49%) | 1.623±0.050 (std x 82.87%) |
| 5°C T3M (1/10) | C1 | 97.40 | 99.49 | 10.33 (97.82%) | 1.474±0.03 (std x 100%) |
| | C2 | 97.28 | 99.50 | 9.72 (97.69%) | 1.23±0.10 (std x 116.3%) |
| 25°C T3M | C1 | 98.10 | 99.55 | 10.41 (92.05%) | 1.267±0.27 (std x 112.1%) |
| (1/10) | C2 | 98.17 | 99.50 | 9.91 (99.60%) | 1.42±0.12 (std x 105%) |
| Wstd | B8 (1.8mg/ml) | 95.19 97.73 | 98.14 96.07 | 1.8165 | 1.811±0.069 1.345±0.048 |

Accordingly, it was confirmed that the sGal-9 protein according to the present invention exhibited superior stability in a lyophilized formulation containing 10 mM histidine acetate buffer (pH 6.0), 4 w/v% mannitol, 2 w/v% trehalose, 0.01 w/v% polysorbate 80, and 5 mg/mL glycine per 10 mg of sGal-9 protein, compared to formulations using other excipients or buffer systems.

[INDUSTRIAL APPLICABILITY]

The present invention relates to a pharmaceutical formulation comprising a recombinant Galectin-9 protein, and can provide a formulation with enhanced stability through the use of a buffer and other excipients.

[SEQUENCE LISTING FREE TEXT]

Sequence:

Sequence Identification Number: 1
Length: 284
Sequence Type: AA
Feature Location/Qualifier:

- REGION, 1..284

> note, sGal-9

- source, 1..284

> mol_type, protein
> organism, synthetic construct

Residues:

MAFSGSQAPY LSPAVPFSGT IQGGLQDGLQ ITVNGTVLSS SGTRFAVNFQ TGFSGNDIAF 60

HFNPRFEDGG YVVCNTRQNG SWGPEERKTH MPFQKGMPFD LCFLVQSSDF KVMVNGILFV 120

QYFHRVPFHR VDTISVNGSV QLSYISFQHP PYPMPFITTI LGGLYPSKSI LLSGTVLPSA 180

QRFHINLCSG NHIAFHLNPR FDENAVVRNT QIDNSWGSEE RSLPRKMPFV RGQSFSVWIL 240

CEAHCLKVAV DGQHLFEYYH RLRNLPTINR LEVGGDIQLT HVQT 284

Sequence Identification Number: 2
Length: 146
Sequence Type: AA
Feature Location/Qualifier:

- REGION, 1..146

> note, CCRD

- source, 1..146

> mol_type, protein
> organism, synthetic construct

Residues:

TPAIPPMMYP HPAYPMPFIT TILGGLYPSK SILLSGTVLP

SAQRFHINLC SGNHIAFHLN 60

PRFDENAVVR NTQIDNSWGS EERSLPRKMP FVRGQSFSVW

ILCEAHCLKV AVDGQHLFEY 120

YHRLRNLPTI NRLEVGGDIQ LTHVQT 146
Sequence Identification Number: 3
Length: 10
Sequence Type: AA
Feature Location/Qualifier:

- REGION, 1..10

> note, CCRD 1-10

- source, 1..10

> mol_type, protein
> organism, synthetic construct

Residues:
TPAIPPMMYP 10

## Claims

1. A pharmaceutical formulation comprising:

   (1) a recombinant stabilized galectin-9 protein;
   (2) a buffer; and
   (3) a stabilizer.

2. The pharmaceutical formulation of claim 1, wherein the recombinant stabilized galectin-9 protein comprises an amino acid sequence represented by SEQ ID NO: 1.

3. The pharmaceutical formulation of claim 1, wherein the recombinant stabilized galectin-9 protein comprises a protein having at least 90% sequence identity to the amino acid sequence represented by SEQ ID NO: 1.

4. The pharmaceutical formulation of claim 1, wherein the recombinant stabilized galectin-9 protein comprises a deletion of the first amino acid residue from the N-terminus of SEQ ID NO: 1.

5. The pharmaceutical formulation of claim 1, wherein the recombinant stabilized galectin-9 protein is present at a concentration of 1 to 20 mg/mL.

6. The pharmaceutical formulation of claim 1, wherein the buffer is one or more selected from the group consisting of citrate, phosphate, histidine, glycine, acetate, tartrate, aspartate, lactate, gluconate, glutamate, succinate, and combinations thereof.

7. The pharmaceutical formulation of claim 1, wherein the buffer is one or more selected from the group consisting of phosphate, histidine, acetate, and combinations thereof.

8. The pharmaceutical formulation of claim 1, wherein the buffer comprises histidine acetate.

9. The pharmaceutical formulation of claim 1, wherein the buffer has a pH of 4.5 to 7.

10. The pharmaceutical formulation of claim 1, wherein the buffer has a pH of 5.5 to 6.5.

11. The pharmaceutical formulation of claim 1, wherein the buffer is present at a concentration of 5 to 20 mM.

12. The pharmaceutical formulation of claim 1, wherein the buffer is present at a concentration of 5 to 15 mM.

13. The pharmaceutical formulation of claim 1, wherein the stabilizer comprises:

   (i) at least one carbohydrate or sugar; and
   (ii) at least one amino acid or a pharmaceutically acceptable salt thereof.

14. The pharmaceutical formulation of claim 13, wherein the carbohydrate or sugar is selected from the group consisting of mannitol, sorbitol, xylitol, maltitol, lactitol, sucrose, trehalose, mannose, maltose, lactose, xylose, ribose, glucose, raffinose, dextran, cyclodextrin, cellobiose, isomaltose, arabinose, glucosamine, and fructose.

15. The pharmaceutical formulation of claim 13, wherein the carbohydrate or sugar is selected from the group consisting of trehalose and mannitol.

16. The pharmaceutical formulation of claim 13, wherein the amino acid is selected from the group consisting of glycine, alanine, serine, threonine, cysteine, valine, leucine, isoleucine, methionine, proline, phenylalanine, tyrosine, tryptophan, aspartic acid, glutamic acid, asparagine, glutamine, histidine, lysine, and arginine.

17. The pharmaceutical formulation of claim 13, wherein the amino acid is glycine.

18. The pharmaceutical formulation of claim 13, wherein each carbohydrate or sugar is present in an amount of 1 to 10 w/v%.

19. The pharmaceutical formulation of claim 13, wherein the carbohydrate or sugar comprises trehalose and mannitol, each in an amount of 1 to 5 w/v%.

20. The pharmaceutical formulation of claim 13, wherein the amino acid or pharmaceutically acceptable salt thereof is present in an amount of 1 to 20 mg/mL.

21. The pharmaceutical formulation of claim 13, wherein the stabilizer further comprises polysorbate.

22. The pharmaceutical formulation of claim 13, wherein the stabilizer further comprises 0.001 to 0.05 w/v% of polysorbate 20, polysorbate 80, or a combination thereof.

23. The pharmaceutical formulation of claim 13, wherein the pharmaceutical formulation is in a liquid or lyophilized dosage form.

24. A vial prefilled with the pharmaceutical formulation of any one of claims 1 to 23.

25. A syringe prefilled with the pharmaceutical formulation of any one of claims 1 to 23.

26. The pharmaceutical formulation according to any one of claims 1 to 23, comprising a therapeutically effective amount thereof, for use in the prevention or treatment of a bone disease.

27. The pharmaceutical formulation according to any one of claims 1 to 23, comprising a therapeutically effective amount thereof, for use in the prevention or treatment of cancer or an autoimmune disease.

28. Use of the pharmaceutical formulation according to any one of claims 1 to 23 for the manufacture of a medicament for the prevention or treatment of a bone disease.

29. Use of the pharmaceutical formulation according to any one of claims 1 to 23 for the manufacture of a medicament for the prevention or treatment of cancer or an autoimmune disease.

30. Use of the pharmaceutical formulation according to any one of claims 1 to 23 in the prevention or treatment of a bone disease.

31. Use of the pharmaceutical formulation according to any one of claims 1 to 23 in the prevention or treatment of cancer or an autoimmune disease.

32. A method for treating a bone disease in a subject comprising administering a therapeutically effective amount of the pharmaceutical formulation according to any one of claims 1 to 23 to the subject.

33. A method for treating cancer or an autoimmune disease in a subject comprising administering a therapeutically effective amount of the pharmaceutical formulation according to any one of claims 1 to 23 to the subject.

Fig. 1

T0        T=45 min        T=45 min

Fig. 2

T0

25C
for 3days

Fig. 3

F1    F2         F1    F2

At 40 ℃ for 2 W        At 25 ℃ for 2 W

Fig. 4a

WBP B201 WST-1 assay on Molt4

B201-20210301-T0
B201-20210302-T0
SG Standard

Fig. 4b

**WBP B201 WST-1 assay on Molt4**

Fig. 4c

**WBP B201 WST-1 assay on Molt4**

Fig. 5

Before FD

After FD

5 minutes after dissolution

30 minutes after dissolution

Fig. 6

Before FD

T0(After FD)

T1M

T2M

Fig. 7

| T0 | T1M | T2M |

M    std.   C1    C2       M   std.   C1    C2      M   std.   C1    C2
10th FD T0              10th FD T1M 37℃         10th FD T2M 37℃

Fig. 8a

Fig. 8b

After FD

Before FD

Fig. 8c

Fig. 8d

【Fig. 9a】

Fig. 9b

**After FD(T0)**

Before FD

Fig. 9c

**T2M, 37°C**

UV-WL 1 280 nm Results

| Peak Number | Name | Retention Time | Area | Area % | Height | Height Percent | Theoretical plates (USP) | Resolution (USP) | Asymmetry (10%) | S/N |
|---|---|---|---|---|---|---|---|---|---|---|
| 1 | | 6.747 | 4713 | 0.07 | 325 | 0.24 | 5498 | 0.000 | 1.450 | 0 |
| 2 | s-Gal 9 | 9.133 | 5373043 | 84.97 | 121969 | 88.34 | 1300 | 3.466 | 2.168 | 52 |
| 3 | | 10.737 | 694733 | 10.99 | 11260 | 8.16 | 164 | 0.735 | 0.000 | 5 |
| 4 | | 12.907 | 198553 | 3.14 | 2194 | 1.59 | 227 | 0.441 | 0.000 | 1 |
| 5 | | 15.687 | 16476 | 0.26 | 565 | 0.41 | 435 | 0.865 | 0.000 | 0 |
| 6 | | 16.380 | 35569 | 0.56 | 1748 | 1.27 | 23329 | 0.404 | 0.000 | 1 |
| Totals | | | 6323087 | 100.00 | 138061 | 100.00 | | | | |

**T1M, 37°C**

UV-WL 1 280 nm Results

| Peak Number | Name | Retention Time | Area | Area % | Height | Height Percent | Theoretical plates (USP) | Resolution (USP) | Asymmetry (10%) | S/N |
|---|---|---|---|---|---|---|---|---|---|---|
| 1 | | 2.790 | 2215 | 0.04 | 106 | 0.08 | 424 | 0.000 | 1.663 | 0 |
| 2 | s-Gal 9 | 8.753 | 5516355 | 99.80 | 125644 | 99.12 | 1252 | 7.785 | 2.063 | 1206 |
| 3 | | 12.190 | 1370 | 0.02 | 178 | 0.14 | 72687 | 5.872 | 0.838 | 2 |
| 4 | | 15.387 | 7531 | 0.14 | 827 | 0.65 | 61622 | 14.910 | 1.003 | 16 |
| Totals | | | 5527471 | 100.00 | 126755 | 100.00 | | | | |

C1

Fig. 9d

C2

T1M, 37°C

UV-WL 1 280 nm Results

| Peak Number | Name | Retention Time | Area | Area % | Height | Height Percent | Theoretical plates (USP) | Resolution (USP) | Asymmetry (10%) | S/N |
|---|---|---|---|---|---|---|---|---|---|---|
| 1 | | 2.753 | 1237 | 0.02 | 58 | 0.04 | 562 | 0.000 | 2.214 | 0 |
| 2 | s-Gal 9 | 8.763 | 5898372 | 99.83 | 133863 | 99.25 | 1190 | 8.117 | 1.988 | 1194 |
| 3 | | 12.193 | 1274 | 0.02 | 199 | 0.15 | 89784 | 5.819 | 0.936 | 3 |
| 4 | | 15.390 | 7562 | 0.13 | 752 | 0.56 | 52534 | 14.821 | 1.120 | 13 |
| Totals | | | 5908445 | 100.00 | 134872 | 100.00 | | | | |

T2M, 37°C

UV-WL 1 280 nm Results

| Peak Number | Name | Retention Time | Area | Area % | Height | Height Percent | Theoretical plates (USP) | Resolution (USP) | Asymmetry (10%) | S/N |
|---|---|---|---|---|---|---|---|---|---|---|
| 1 | | 6.710 | 6328 | 0.10 | 419 | 0.30 | 4689 | 0.000 | 1.313 | 0 |
| 2 | s-Gal 9 | 9.130 | 5413576 | 87.10 | 123163 | 89.54 | 1305 | 3.450 | 2.107 | 63 |
| 3 | | 10.723 | 642078 | 10.33 | 10668 | 7.76 | 124 | 0.655 | 0.000 | 5 |
| 4 | | 12.897 | 138092 | 2.22 | 1880 | 1.37 | 316 | 0.643 | 0.000 | 1 |
| 5 | | 15.723 | 1796 | 0.03 | 129 | 0.09 | 14891 | 1.654 | 0.000 | 0 |
| 6 | | 16.383 | 13702 | 0.22 | 1293 | 0.94 | 56111 | 1.667 | 1.257 | 1 |
| Totals | | | 6215572 | 100.00 | 137552 | 100.00 | | | | |

Fig. 10a

Fig. 10b

# EP 4 663 183 A1

## INTERNATIONAL SEARCH REPORT

| International application No. |
| --- |
| **PCT/KR2024/001962** |

| A. | CLASSIFICATION OF SUBJECT MATTER |
| --- | --- |

**A61K 9/08**(2006.01)i; **A61K 47/22**(2006.01)i; **A61K 47/26**(2006.01)i; **A61K 47/18**(2006.01)i; **A61K 47/12**(2006.01)i; **A61K 47/02**(2006.01)i; **A61K 9/19**(2006.01)i; **A61K 38/17**(2006.01)i; **A61P 19/08**(2006.01)i; **A61P 35/00**(2006.01)i

According to International Patent Classification (IPC) or to both national classification and IPC

| B. | FIELDS SEARCHED |
| --- | --- |

Minimum documentation searched (classification system followed by classification symbols)

A61K 9/08(2006.01); A23L 33/17(2016.01); A61K 35/12(2006.01); A61K 38/17(2006.01); A61K 39/395(2006.01); A61P 19/02(2006.01); C07K 14/47(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 갈렉틴 9 단백질(galectin 9 protein), 완충제(buffer), 안정화제(stabilizer), 재조합(recombination), 골질환(bone disease), 자가면역질환(autoimmune disease)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | KR 10-2012-0126130 A (GALPHARMA CO., LTD.) 20 November 2012 (2012-11-20)<br>See abstract; claim 1; paragraphs [0030], [0033], [0035], [0039], [0044], [0061], [0128]-[0133], [0169], [0213], [0238] and [0334]; and SEQ ID NO: 2. | 1-31 |
| A | KR 10-2022-0068158 A (GBIOLOGICS INC.) 25 May 2022 (2022-05-25)<br>See entire document. | 1-31 |
| A | KR 10-2005-0103474 A (GALPHARMA CO., LTD.) 31 October 2005 (2005-10-31)<br>See entire document. | 1-31 |
| A | US 2015-0307574 A1 (NATIONAL UNIVERSITY CORPORATION KAGAWA UNIVERSITY) 29 October 2015 (2015-10-29)<br>See entire document. | 1-31 |
| A | KR 10-2267413 B1 (GBIOLOGICS INC.) 21 June 2021 (2021-06-21)<br>See entire document. | 1-31 |

☐ Further documents are listed in the continuation of Box C.   ☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **05 June 2024** | **05 June 2024** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

44

## INTERNATIONAL SEARCH REPORT

| International application No. |
| --- |
| **PCT/KR2024/001962** |

| Box No. I | Nucleotide and/or amino acid sequence(s) (Continuation of item 1.c of the first sheet) |
| --- | --- |

1.  With regard to any nucleotide and/or amino acid sequence disclosed in the international application, the international search was carried out on the basis of a sequence listing:

   a.  ☑ forming part of the international application as filed.

   b.  ☐ furnished subsequent to the international filing date for the purposes of international search (Rule 13*ter*.1(a)),

   ☐ accompanied by a statement to the effect that the sequence listing does not go beyond the disclosure in the international application as filed.

2.  ☐ With regard to any nucleotide and/or amino acid sequence disclosed in the international application, this report has been established to the extent that a meaningful search could be carried out without a WIPO Standard ST.26 compliant sequence listing.

3.  Additional comments:

Form PCT/ISA/210 (continuation of first sheet) (July 2022)

**INTERNATIONAL SEARCH REPORT**

| International application No. |
| --- |
| **PCT/KR2024/001962** |

---

**Box No. II      Observations where certain claims were found unsearchable (Continuation of item 2 of first sheet)**

This international search report has not been established in respect of certain claims under Article 17(2)(a) for the following reasons:

1. ☑ Claims Nos.: **32, 33**
   because they relate to subject matter not required to be searched by this Authority, namely:

   Claims 32 and 33 pertain to a method for treatment of the human body, and thus pertain to a subject matter on which the International Searching Authority is not required to carry out an international search under the provisions of PCT Article 17(2)(a)(i) and PCT Rule 39.1(iv).

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

---

Form PCT/ISA/210 (continuation of first sheet) (July 2022)

EP 4 663 183 A1

## INTERNATIONAL SEARCH REPORT
### Information on patent family members

International application No.

**PCT/KR2024/001962**

| Patent document cited in search report | | | Publication date (day/month/year) | Patent family member(s) | | | Publication date (day/month/year) |
|---|---|---|---|---|---|---|---|
| KR | 10-2012-0126130 | A | 20 November 2012 | CA | 2561696 | A1 | 06 October 2005 |
| | | | | CA | 2561696 | C | 03 September 2013 |
| | | | | CN | 104292321 | A | 21 January 2015 |
| | | | | CN | 1957082 | A | 02 May 2007 |
| | | | | CN | 1957082 | B | 17 September 2014 |
| | | | | DK | 1736541 | T3 | 06 May 2013 |
| | | | | EP | 1736541 | A1 | 27 December 2006 |
| | | | | EP | 1736541 | B1 | 23 January 2013 |
| | | | | ES | 2407465 | T3 | 12 June 2013 |
| | | | | JP | 4792390 | B2 | 12 October 2011 |
| | | | | KR | 10-1222281 | B1 | 28 January 2013 |
| | | | | KR | 10-2007-0031887 | A | 20 March 2007 |
| | | | | PT | 1736541 | E | 31 January 2013 |
| | | | | US | 2010-0203628 | A1 | 12 August 2010 |
| | | | | US | 2013-0017998 | A1 | 17 January 2013 |
| | | | | US | 8268324 | B2 | 18 September 2012 |
| | | | | US | 8580743 | B2 | 12 November 2013 |
| | | | | WO | 2005-093064 | A1 | 06 October 2005 |
| KR | 10-2022-0068158 | A | 25 May 2022 | CA | 3199417 | A1 | 27 May 2022 |
| | | | | CN | 116615244 | A | 18 August 2023 |
| | | | | JP | 2023-550415 | A | 01 December 2023 |
| | | | | US | 2023-0414712 | A1 | 28 December 2023 |
| | | | | WO | 2022-108243 | A1 | 27 May 2022 |
| KR | 10-2005-0103474 | A | 31 October 2005 | AU | 2003-262266 | A1 | 13 August 2004 |
| | | | | CA | 2514108 | A1 | 05 August 2004 |
| | | | | CN | 1744910 | A | 08 March 2006 |
| | | | | EP | 1586325 | A1 | 19 October 2005 |
| | | | | US | 2006-0134119 | A1 | 22 June 2006 |
| | | | | WO | 2004-064857 | A1 | 05 August 2004 |
| US | 2015-0307574 | A1 | 29 October 2015 | JP | 5888761 | B2 | 22 March 2016 |
| | | | | US | 9908921 | B2 | 06 March 2018 |
| | | | | WO | 2014-080703 | A1 | 30 May 2014 |
| KR | 10-2267413 | B1 | 21 June 2021 | BR | 112022005782 | A2 | 11 October 2022 |
| | | | | CA | 3157954 | A1 | 20 May 2021 |
| | | | | CN | 115605217 | A | 13 January 2023 |
| | | | | KR | 10-2021-0056927 | A | 20 May 2021 |

Form PCT/ISA/210 (patent family annex) (July 2022)

**REFERENCES CITED IN THE DESCRIPTION**

**Patent documents cited in the description**

- KR 1020220068158